(19) Europäisches Patentamt
European Patent Office
Office européen des brevets

(11) Veröffentlichungsnummer: **0 603 786 A2**

# (12) EUROPÄISCHE PATENTANMELDUNG

(21) Anmeldenummer: **93120516.5**

(22) Anmeldetag: **20.12.93**

(51) Int. Cl.5: **C09K 19/20**, C09K 19/30, C09K 19/34, C09K 19/40, C09K 19/42, C09K 19/46, C09K 19/58

(30) Priorität: **23.12.92 DE 4243705**

(43) Veröffentlichungstag der Anmeldung: **29.06.94 Patentblatt 94/26**

(84) Benannte Vertragsstaaten: **DE FR GB**

(71) Anmelder: **HOECHST AKTIENGESELLSCHAFT**
**Brüningstrasse 50**
**D-65929 Frankfurt am Main(DE)**

(72) Erfinder: **Hornung, Barbara**
**Schulstrasse 21a**
**D-63594 Haselroth(DE)**
Erfinder: **Jungbauer, Dietmar, Dr.**
**Thomas-Mann-Strasse 8**
**D-64331 Weiterstadt(DE)**
Erfinder: **Manero, Javier, Dr.**
**Sindlinger Bahnstrasse 163**
**D-65931 Frankfurt am Main(DE)**

(54) **Alkylsubstituierte Hydrochinonderivate zur Verwendung in ferroelekfrischen Flüssigkristallmischungen.**

(57) Ferroelektrische Flüssigkristallmischung, enthaltend mindestens eine Verbindung der Formel I,

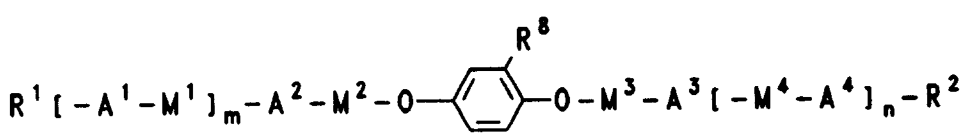

(I)

in der die Symbole und Indizes folgende Bedeutung haben:

$R^8$ ist Alkyl oder Halogen und

$R^1[-A^1-M^1]_m-A^2-M^2$ $M^3-A^3[-M^4-A^4]_n-R^2$ sind mesogene Reste.

Die Verbindungen der Formel I induzieren schon in geringen Mengen nematische Phasen, lassen aber wichtige Eigenschaften der $S_C^*$ Phase, wie Viskosität und Schaltwinkel, nahezu unbeeinflußt.

EP 0 603 786 A2

Flüssigkristalle haben insbesondere im letzten Jahrzehnt Eingang in verschiedene technische Gebiete gefunden, in denen elektrooptische und Anzeigevorrichtungs-Eigenschaften gefragt sind (z.B. in Uhren-, Taschenrechner-und Schreibmaschinenanzeigen). Diese Anzeigevorrichtungen beruhen auf den dielektrischen Ausrichtungseffekten in den nematischen, cholesterischen und/oder smektischen Phasen der flüssigkristallinen Verbindungen, wobei - verursacht durch die dielektrische Anisotropie - die molekulare Längsachse der Verbindungen eine bevorzugte Ausrichtung in einem angelegten elektrischen Feld einnimmt. Die üblichen Schaltzeiten bei diesen Anzeigevorrichtungen sind für viele andere potentielle Anwendungsgebiete von Flüssigkristallen zu lang. Dieser Nachteil macht sich insbesondere dann bemerkbar, wenn eine große Anzahl von Bildpunkten angesteuert werden muß. Die Herstellungskosten von Geräten, die größere Bildschirmflächen enthalten, sind dann im allgemeinen zu hoch.

Neben den nematischen und cholesterischen Flüssigkristallen haben seit einigen wenigen Jahren in zunehmendem Maße auch optisch aktive smektische (ferroelektrische) Flüssigkristall-Phasen an Bedeutung gewonnen.

Clark und Lagerwall konnten zeigen, daß die Verwendung ferroelektrischer Flüssigkristallsysteme in sehr dünnen Zellen zu elektrooptischen Schalt-oder Anzeigeelementen führt, die im Vergleich zu den herkömmlichen TN ("twisted nematic")-Zellen um bis zu einem Faktor 1000 schnellere Schaltzeiten aufweisen (vgl. z.B. Lagerwall et all. "Ferroelectric liquid Crystals for Displays", SID Symposium, October Meeting 1985, San Diego, Cal., USA). Aufgrund dieser und anderer günstiger Eigenschaften, z.B. der bistabilen Schaltmöglichkeit und des nahezu blickwinkelunabhängigen Kontrasts, sind FLCs grundsätzlich für die obengenannten Anwendungsgebiete, z.B. über eine Matrixansteuerung, gut geeignet. Auch auf dem Gebiet der räumlichen Lichtmodulatoren (vgl. z.B. U.Efron in "Spatial Light Modulators and Applications", SPIE vol. 1150, S. 46 ff.) sind ferroelektrische Flüssigkristalle wegen ihres hohen Kontrasts und ihrer Geschwindigkeit besonders geeignet. Weiterhin lassen sie sich auf dem Gebiet der nichtlinearen Optik (NLO) einsetzen (siehe z.B. Mol. Cryst Liq.Cryst. (1991) 198, 51-60; Ferroelectrics (1991)121 , 247-257 und SPIE Vol. 1080 Liq.Cryst.Chem.,Physics and Appl. (1989)).

Für die Anwendung in Lichtventilen ist eine gleichmäßige Verteilung der Orientierung über die gesamte Lichtventilfläche wünschenswert, um bei geeigneter Stellung von Polarisator und/oder Analysator maximalen, gleichmäßigen Kontrast und Helligkeit über der gesamten Fläche zu erhalten. Ein Weg, eine solche homogene Orietierung zu erreichen, ist, Mischungen mit einer bestimmten Phasenfolge in Kombination mit Orientierungsschichten zu verwenden (H. Olnishi et al. "Fast switching ferroelectric liquid crystal mixture...." , 2320 Proceedings of Society for Information Display (SID), 28 (1987) No. 2, New York, NY, USA). Dabei wird die molekulare Ordnung der ferroelektrischen schaltbaren chiralen smektischen C-Phase nicht direkt beim Abkühlen aus der isotropen Phase erhalten, sondern über die nematische und die smektische A Flüssigkristallphase. Im allgemeinen werden bei der Phasenfolge $S_c^*$ , $S_A$, N*, I die besten Orientierungen erreicht, sofern die nematische Phase in der Zelle uniform homogen orientiert vorliegt. Da einzelne Reinsubstanzen nicht alle diese gewünschten Eigenschaften erfüllen, werden üblicherweise Mischungen erstellt, die die gewünschte Phasenfolge bieten. Da das Hauptaugenmerk bei der Entwicklung jedoch auf die Eigenschaften der schaltbaren $S_C^*$ Phase zu richten ist, muß die Induktion der $S_A$ bzw. N* Phase als notwendiges Übel zur besseren Orientierung betrachtet werden. Es gilt daher, Moleküle zu finden, die die gewünschte/n Phase/n leicht, d.h. schon in geringen Konzentrationen induzieren.

Die Molekülstruktur der für obengenannte Anwendungsgebiete in Mischungen einsetzbaren Flüssigkristalle baut im wesentlichen auf para- bzw. 1,4-substituierten aromatischen und gesättigten Sechsringen, wie 1,4-Phenylen, Pyridin-2,5-diyl, Pyrimidin-2,5-diyl und trans-1,4-Cyclohexylen auf, die durch Verknüpfung untereinander, entweder direkt oder über geeignete Zwischenstücke, sowie durch Verknüpfung mit geeigneten terminalen Gruppen die aus zahlreichen Beispielen bekannten (siehe z.B.: D.Demus, H.Zachke, "Flüssige Kristalle in Tabellen I und II, VEB Deutscher Verlag für Grundstoffindustrie, Leipzig 1974 und 1984) langgestreckten "stäbchenförmigen" Moleküle liefern.

Es zeigte sich nun überraschenderweise, daß alkylsubstituierte Hydrochinonderivate auch in FLC Mischungen schon in sehr geringen Anteilen nematische Phasen induzieren oder verbreitern können aber wichtige Eigenschaften der $S_C^*$ Phase, wie Schaltzeit (Viskosität) und Schaltwinkel, nur wenig beeinflussen.

Vertreter dieser Substanzklasse sind teilweise bekannt (siehe z.B. JACS 96 (1975) 1585 oder Mol. Cryst. Liq. Cryst. 61(1980) 229 oder DD 2348-193, DD 107563, haben aber bisher in der Entwicklung ferroelektrischer Flüssigkristallmischungen keine Beachtung gefunden, da sie sehr breite nematische Phasen aber kaum smektische aufweisen und der Fachmann davon ausgehen mußte, daß zwar die Induktion der nematischen Phase in FLC Mischungen möglich sei, aber gleichzeitig die Phasenlage, insbesondere der smektischen Phasen, sehr ungünstig beeinflußt würde. Zwar wird auch der $S_C$-$S_A$ Phasenübergang durch Zugabe der Substanz herabgesetzt, dies kann aber auch von großem Vorteil sein.

Gegenstand der vorliegenden Erfindung ist daher eine ferroelektrische Flüssigkristallmischung, enthaltend mindestens eine Verbindung der Formel I,

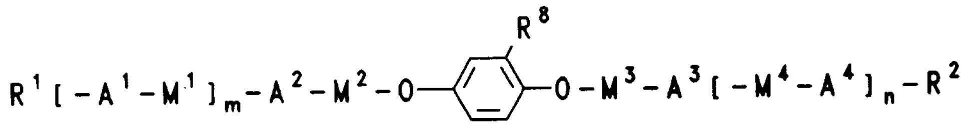

(I)

in der die Symbole und Indizes folgende Bedeutung haben:

$R^1$, $R^2$ sind gleich oder verschieden Wasserstoff oder ein geradkettiger oder verzweigter Alkylrest mit 1 bis 20 C-Atomen (mit oder ohne asymmetrische C-Atome), wobei auch eine oder mehrere $CH_2$-Gruppen durch - O-, -S-, -CO-, -CS-, -C≡C-, Δ, -Si$(CH_3)_2$-, 1,4-Phenylen, trans-1,4-Cyclohexylen oder trans-1,3-Cyclopentylen ersetzt sein können, mit der Maßgabe, daß Sauerstoffatome und/oder Schwefelatome nicht unmittelbar miteinander gebunden sein dürfen, und/oder ein oder mehrere H-Atome des Alkylrestes durch -F, -Cl, -Br, -$OR^3$, -SCN, -OCH oder - $N_3$ substituiert sein können, oder eine der nachfolgenden chiralen Gruppen:

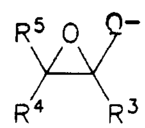
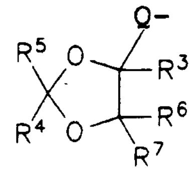
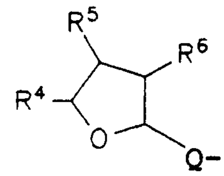

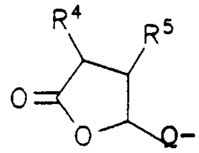
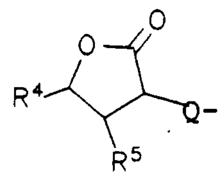
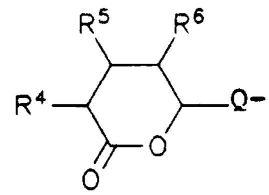

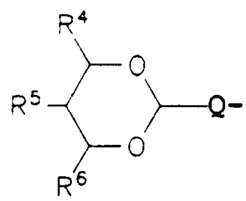
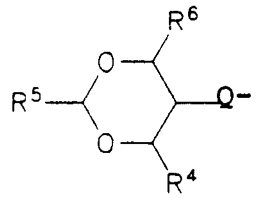
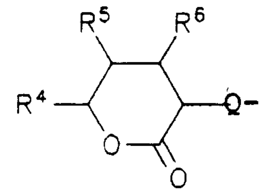

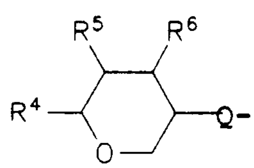
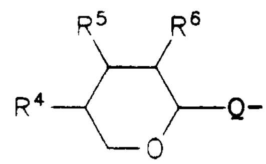
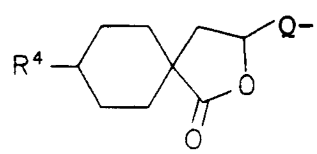

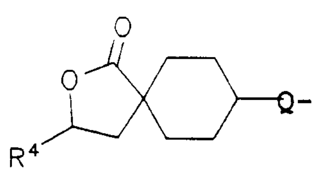
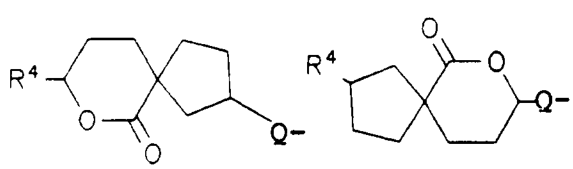

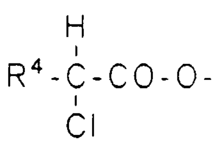
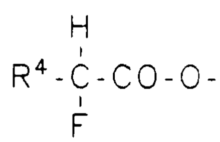
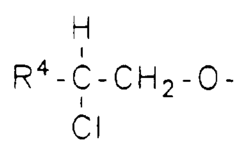
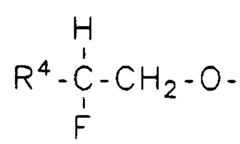

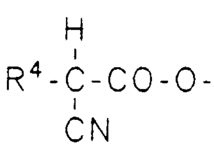
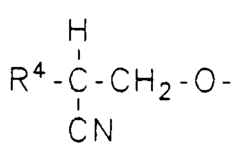
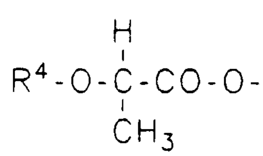
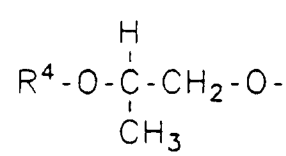

$R^3$, $R^4$, $R^5$, $R^6$, $R^7$ sind gleich oder verschieden Wasserstoff oder ein geradkettiger oder verzweigter Alkylrest mit 1-16 C-Atomen (mit oder ohne asymmetrische C-Atome), wobei auch eine oder mehrere $CH_2$-Gruppen durch -O- oder - CH = CH- ersetzt sein können, mit der Maßgabe, daß Sauerstoffatome nicht unmittelbar miteinander gebunden sein dürfen, und/oder ein oder mehrere H-Atome des Alkylrestes durch - F oder -Cl substituiert sein können; $R^4$ und $R^5$ können zusammen auch $-(CH_2)_4$- oder $-(CH_2)_5$- sein, wenn sie an ein Oxiran-, Dioxolan-, Tetrahydrofuran-, Tetrahydropy-

$-(CH_2)_5-$ sein, wenn sie an ein Oxiran-, Dioxolan-, Tetrahydrofuran-, Tetrahydropyran-oder Valerolacton-System gebunden sind;

$R^8$ ist $R^3$, Brom, Chlor, Fluor, $-NO_2$, -SCN oder - OCN;

Q ist -CO-O-, -O-CO-, $-CH_2-O-$, $-O-CH_2-$ oder - O-CO-O-;

$M^1$, $M^4$ sind gleich oder verschieden -O-, -S-, -CO-, -CO-O-, -O-CO-, -O-CO-O-, $-CH_2-O-$, $-O-CH_2-$, -CH = CH-, - C≡C- oder eine Einfachbindung;

$M^2$, $M^3$ sind gleich oder verschieden -CO-, -CO-O-, oder $-CH_2-$;

$A^1$, $A^2$, $A^3$, $A^4$ sind gleich oder verschieden 1,2-Phenylen, wobei ein oder mehrere H-Atome durch F, Cl und/oder CN ersetzt sein können, 1,3-Phenylen, wobei ein oder mehrere H-Atome durch F, Cl und/oder CN ersetzt sein können, 1,4-Phenylen, wobei ein oder mehrere H-Atome durch F, Cl und/oder CN ersetzt sein können, Pyrazin-2,3-diyl, wobei ein oder zwei H-Atome durch F, Cl und/oder CN ersetzt sein können, Pyrazin-2,5-diyl, wobei ein oder zwei H-Atome durch F, Cl und/oder CN ersetzt sein können, Pyrazin-2,6-diyl, wobei ein oder zwei H-Atome durch F, Cl und/oder CN ersetzt sein können, Pyridazin-3,4-diyl, wobei ein oder zwei H-Atome durch F, Cl und/oder CN ersetzt sein können, Pyridazin-3,5-diyl, wobei ein oder zwei H-Atome durch F, Cl und/oder CN ersetzt sein können, Pyridazin-3,6-diyl, wobei ein oder zwei H-Atome durch F, Cl und/oder CN ersetzt sein können, Pyridin-2,3-diyl, wobei ein oder mehrere H-Atome durch F, Cl und/oder CN ersetzt sein können, Pyridin-2,4-diyl, wobei ein oder mehrere H-Atome durch F, Cl und/oder CN ersetzt sein können, Pyridin-2,5-diyl, wobei ein oder mehrere H-Atome durch F, Cl und/oder CN ersetzt sein können, Pyridin-2,6-diyl, wobei ein oder mehrere H-Atome durch F, Cl und/oder CN ersetzt sein können, Pyrimidin-2,5-diyl, wobei ein oder zwei H-Atome durch F, Cl und/oder CN ersetzt sein können, Pyrimidin-2,6-diyl, wobei ein oder zwei H-Atome durch F, Cl und/oder CN ersetzt sein können, 1,2-Cyclohexylen, bei dem ein oder mehrere H-Atome durch CN, $CH_3$, F und oder $CF_3$ ersetzt sein können, 1,3-Cyclohexylen, bei dem ein oder mehrere H-Atome durch CN, $CH_3$, F und/oder $CF_3$ ersetzt sein können, 1 ,4-Cyclohexylen, bei dem ein oder mehrere H-Atome durch CN, $CH_3$, F und/oder $CF_3$ ersetzt sein können, (1,3,4)-Thiadiazol-2,5-diyl, 1,3-Dioxan-2,5-diyl, 1,3-Dithian-2,5-diyl, 1,3-Thiazol-2,4-diyl, wobei ein H-Atom durch F, Cl und/oder CN ersetzt sein kann, 1,3-Thiazol-1,5-diyl, wobei ein H-Atome durch F, Cl und/oder CN ersetzt sein kann, Thiophen-2,4-diyl, wobei ein oder zwei H-Atome durch F, Cl und/oder CN ersetzt sein können, Thiophen-2,5-diyl, wobei ein oder zwei H-Atome durch F, Cl und/oder CN ersetzt sein können, Piperazin-1,4-diyl, Piperazin-2,5-diyl, Naphthalin-2,6-diyl, wobei ein oder mehrere H-Atome durch F, Cl und/oder CN ersetzt sein können, Naphthalin-2,7-diyl, wobei ein oder mehrere H-Atome durch F, Cl und/oder CN ersetzt sein können, Bicyclo[2.2.2]octan-1,4-diyl oder 1,3-Dioxaborinan-2,5-diyl;

m, n sind null oder eins.

Bevorzugt sind Verbindungen der allgemeinen Formel I, in der die Symbole und Indizes folgende Bedeutung haben:

$R^1$, $R^2$ sind gleich oder verschieden Wasserstoff oder ein geradkettiger oder verzweigter Alkylrest mit 1 bis 18 C-Atomen (mit oder ohne asymmetrische C-Atome), wobei auch eine oder mehrere $CH_2$-Gruppen durch - O-, -CO-, -C≡C-, Δ, $-Si(CH_3)_2-$ oder trans-1,4-Cyclohexylen ersetzt sein können, mit der Maßgabe, daß Sauerstoffatome nicht unmittelbar miteinander gebunden sein dürfen, und/oder ein oder mehrere H-Atome des Alkylrestes durch - F, -Cl, $-OR^3$, -OCN oder $-N_3$ substituiert sein können, oder eine der nachfolgenden chiralen Gruppen:

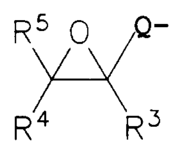 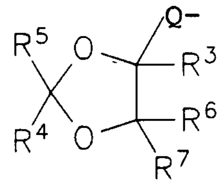 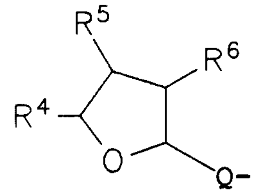

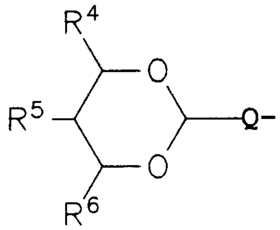 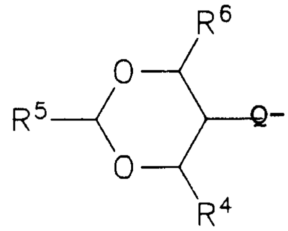 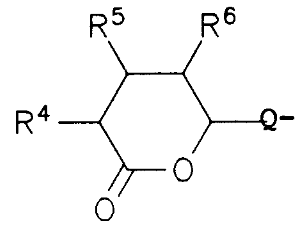

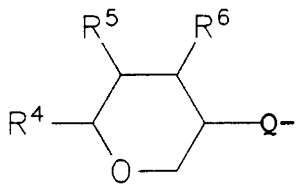 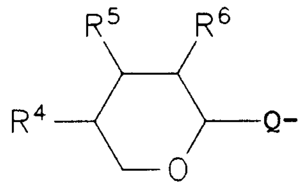 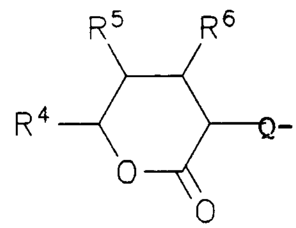

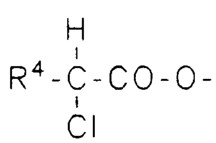 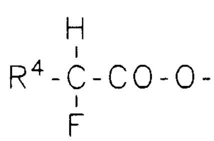 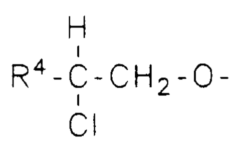 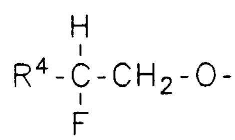

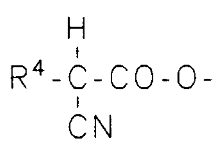 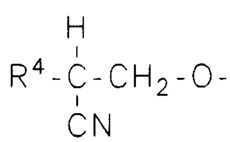 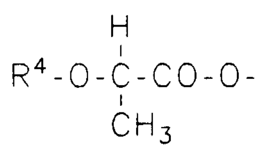 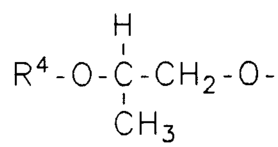

| | |
|---|---|
| $R^3$, $R^4$, $R^5$, $R^6$, $R^7$ | sind gleich oder verschieden Wasserstoff oder ein geradkettiger oder verzweigter Alkylrest mit 1-16 C-Atomen (mit oder ohne asymmetrische C-Atome), wobei auch eine oder mehrere $CH_2$-Gruppen durch -O- oder - CH=CH- ersetzt sein können, mit der Maßgabe, daß Sauerstoffatome nicht unmittelbar miteinander gebunden sein dürfen, und/oder ein oder mehrere H-Atome des Alkylrestes durch - F oder -Cl substituiert sein können; $R^4$ und $R^5$ können zusammen auch $-(CH_2)_4-$ oder $-(CH_2)_5-$ sein, wenn sie an ein Oxiran-, Dioxolan-, Tetrahydrofuran-, Tetrahydropyran-oder Valerolacton-System gebunden sind; |
| $R^8$ | ist $R^3$, Brom, Chlor, Fluor, $-NO_2$, -SCN oder - OCN; |
| Q | ist -CO-O-, -O-CO-, $-CH_2-O-$, $-O-CH_2-$ oder - O-CO-O-; |
| $M^1$, $M^4$ | sind gleich oder verschieden -O-, -CO-, -CO-O-, -O-CO-, -O-CO-O-, -O-CS-O-, $-CH_2-O-$, $-O-CH_2-$, -CH=CH-, -C≡C-oder eine Einfachbindung; |
| $M^2$, $M^3$ | sind gleich oder verschieden -CO-, -CO-O- oder $-CH_2-$; |
| $A^1$, $A^2$, $A^3$, $A^4$ | sind gleich oder verschieden 1,3-Phenylen, wobei ein oder mehrere H-Atome durch F, Cl und/oder CN ersetzt sein können, 1,4-Phenylen, wobei ein oder mehrere H-Atome durch F, Cl und/oder CN ersetzt sein können, Pyrazin-2,5-diyl, wobei ein oder zwei H-Atome durch F, Cl und/oder CN ersetzt sein können, Pyrazin-2,6-diyl, wobei ein oder zwei H-Atome durch F, Cl und/oder CN ersetzt |

sein können, Pyridazin-3,5-diyl, wobei ein oder zwei H-Atome durch F, Cl und/oder CN ersetzt sein können, Pyridazin-3,6-diyl, wobei ein oder zwei H-Atome durch F, Cl und/oder CN ersetzt sein können, Pyridin-2,5-diyl, wobei ein oder mehrere H-Atome durch F, Cl und/oder CN ersetzt sein können, Pyridin-2,6-diyl, wobei ein oder mehrere H-Atome durch F, Cl und/oder CN ersetzt sein können, Pyrimidin-2,5-diyl, wobei ein oder zwei H-Atome durch F, Cl und/oder CN ersetzt sein können, Pyrimidin-2,6-diyl, wobei ein oder zwei H-Atome durch F, Cl und/oder CN ersetzt sein können, 1,2-Cyclohexylen, bei dem ein oder mehrere H-Atome durch CH, $CH_3$, F und oder $CF_3$ ersetzt sein können, cis-1,3-Cyclohexylen, bei dem ein oder mehrere H-Atome durch CH, $CH_3$, F und/oder $CF_3$ ersetzt sein können, trans-1,4-Cyclohexylen, bei dem ein oder mehrere H-Atome durch CN, $CH_3$, F und/oder $CF_3$ ersetzt sein können, (1,3,4)-Thiadiazol-2,5-diyl, 1,3-Dioxan-2,5-diyl, 1,3-Dithian-2,5-diyl, 1,3-Thiazol-2,4-diyl, 1,3-Thiazol-2,5-diyl, wobei ein H-Atom durch F, Cl und/oder CN ersetzt sein kann, Thiophen-2,4-diyl, wobei ein oder zwei H-Atome durch F, Cl und/oder CN ersetzt sein können, Thiophen-2,5-diyl, wobei ein oder zwei H-Atome durch F, Cl und/oder CN ersetzt sein können, Naphthalin-2,6-diyl, wobei ein oder mehrere H-Atome durch F, Cl und/oder CN ersetzt sein können, Naphthalin-2,7-diyl, wobei ein oder mehrere H-Atome durch F, Cl und/oder CN ersetzt sein können oder 1,3-Dioxaborinan-2,5-diyl;

m, n sind null oder eins.

Insbesondere bevorzugt sind Verbindungen der allgemeinen Formel I, in der die Symbole und Indizes folgende Bedeutung haben:

$R^1$, $R^2$ sind gleich oder verschieden Wasserstoff oder ein geradkettiger oder verzweigter Alkylrest mit 1 bis 16 C-Atomen (mit oder ohne asymmetrische C-Atome), wobei auch eine, zwei oder drei $-CH_2$-Gruppen durch -O-,-CO-, Δ, $-Si(CH_3)_2$- oder trans-1,4-Cyclohexylen ersetzt sein können, mit der Maßgabe, daß Sauerstoffatome nicht unmittelbar miteinander gebunden sein dürfen, und/oder ein oder mehrere H-Atome des Alkylrestes durch - F, -Cl oder $-OR^3$ substituiert sein können, oder eine der nachfolgenden chiralen Gruppen:

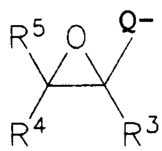
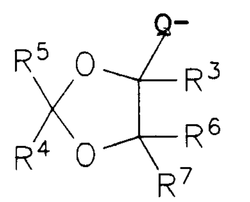
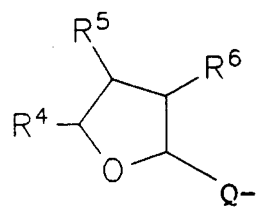

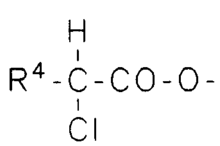
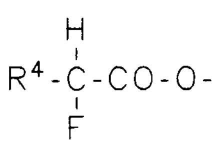
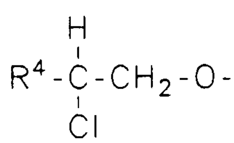
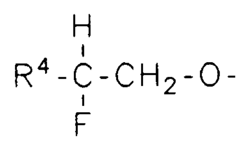

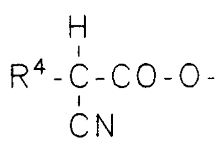
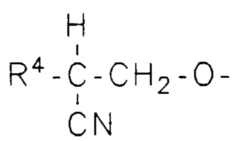
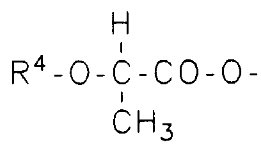
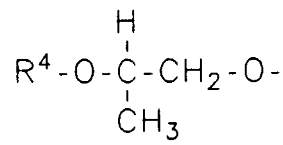

$R^3$, $R^4$, $R^5$, $R^6$, $R^7$ sind gleich oder verschieden Wasserstoff oder ein geradkettiger oder verzweigter Alkylrest mit 1 bis 14 C-Atomen (mit oder ohne asymmetrische C-Atome), wobei auch eine oder mehrere $-CH_2$-Gruppen durch - O- oder -CH=CH- ersetzt sein können, mit der Maßgabe, daß Sauerstoffatome nicht unmittelbar miteinander gebunden sein dürfen, und/oder ein oder mehrere H-Atome des Alkylrestes durch -F oder -Cl substituiert sein können; $R^4$ und $R^5$ können zusammen auch $-(CH_2)_4$-

oder - $(CH_2)_5$- sein, wenn sie an ein Oxiran- oder Dioxolan-System gebunden sind;

$R^8$ ist $R^3$, Brom, Cl, F, $-NO_2$, -SCN oder - OCN;

Q ist -CO-O-oder $-CH_2$-O-;

$M^1$, $M^4$ sind gleich oder verschieden -O-,-CO-, -CO-O-, -O-CO-, -O-CO-O-, $-CH_2$-O-, -O-$CH_2$-, -CH = CH-, oder eine Einfachbindung;

$M^2$, $M^3$ sind gleich oder verschieden -CO-, -CO-O-, $-CH_2$-;

$A^1$, $A^2$, $A^3$, $A^4$ sind gleich oder verschieden 1,4-Phenylen, wobei ein oder zwei H-Atome durch F, Cl und/oder CN ersetzt sein können, Pyrazin-2,5-diyl, wobei ein oder zwei H-Atome durch F, Cl und/oder CN ersetzt sein können, Pyridazin-3,6-diyl, wobei ein oder zwei H-Atome durch F, Cl und/oder CN ersetzt sein können, Pyridin-2,5-diyl, wobei ein oder mehrere H-Atome durch F, Cl und/oder CN ersetzt sein können, Pyrimidin-2,5-diyl, wobei ein oder zwei H-Atome durch F, Cl und/oder CN ersetzt sein können, trans-1,4-Cyclohexylen, bei dem ein oder zwei H-Atome durch CN, $CH_3$, F und/oder $CF_3$ ersetzt sein können, (1,3,4)-Thiadiazol-2,5-diyl, 1,3-Dithian-2,5-diyl, 1,3-Thiazol-2,4-diyl, 1,3-Thiazol-2,5-diyl, Thiophen-2,5-diyl, wobei ein oder zwei H-Atome durch F, Cl und/oder CN ersetzt sein können, Naphthalin-2,6-diyl, wobei ein oder mehrere H-Atome durch F, Cl und/oder CN ersetzt sein können oder 1,3-Dioxan-2,5-diyl;

m, n sind null oder eins mit der Bedingung, daß die Summe m + n kleiner als zwei ist.

Ganz besonders bevorzugt sind Verbindungen der allgemeinen Formel I, in der die Symbole und Indizes folgende Bedeutung haben:

$R^1$, $R^2$ sind gleich oder verschieden Wasserstoff oder ein geradkettiger oder verzweigter Alkylrest mit 1 bis 16 C-Atomen (mit oder ohne asymmetrische C-Atome), wobei auch eine, zwei oder drei $CH_2$-Gruppen durch -O-, -CO-, $\Delta$, $-Si(CH_3)_2$- oder trans-1,4-Cyclohexylen ersetzt sein können, mit der Maßgabe, daß Sauerstoffatome nicht unmittelbar miteinander gebunden sein dürfen, und/oder ein oder mehrere H-Atome des Alkylrestes durch - F oder $-OR^3$ substituiert sein können oder eine der nachfolgenden chiralen Gruppen:

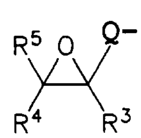

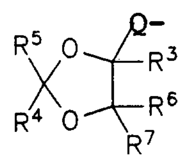

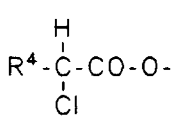

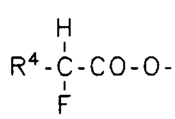

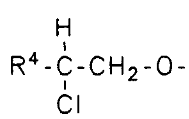

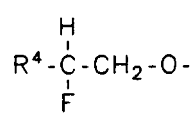

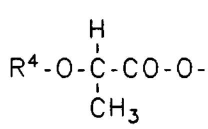

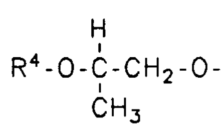

$R^3$, $R^4$, $R^5$, $R^6$, $R^7$ sind gleich oder verschieden Wasserstoff oder ein geradkettiger oder verzweigter Alkylrest mit 1 bis 10 C-Atomen (mit oder ohne asymmetrische C-Atome), wobei auch eine oder zwei $CH_2$-Gruppen durch - O- oder -CH = CH-ersetzt sein können, mit der Maßgabe, daß Sauerstoffatome nicht unmittelbar miteinander gebunden sein dürfen, und/oder ein oder mehrere H-Atome des Alkylrestes durch - F oder -Cl substituiert sein können; $R^4$ und $R^5$ können zusammen auch $-(CH_2)_4$- oder $-(CH_2)_5$- sein, wenn sie an ein Dioxolan-System gebunden sind;

$R^8$ ist Methyl, Methoxy, Trifluormethyl, Trifluormethoxy, Ethyl, Ethoxy, 1-Propyl, 1-Propyloxy, 2-Propyl, 2-Propyloxy, Acetyl, Trifluoracetyl, $NO_2$, Brom, Chlor oder Fluor;

Q ist -CO-O- oder $-CH_2-O-$;

$M^1$, $M^4$ sind gleich oder verschieden -O-,-CO-, -CO-O-, -O-CO-, -O-CO-O-, $-CH_2-O-$, $-O-CH_2-$, oder eine Einfachbindung;

$M^2$, $M^3$ sind gleich oder verschieden -CO- oder $-CH_2-$;

$A^1$, $A^2$, $A^3$, $A^4$ sind gleich oder verschieden 1,4-Phenylen, wobei ein oder zwei H-Atome durch F, Cl und/oder CN ersetzt sein können, Pyrazin-2,5-diyl, Pyridazin-3,6-diyl, Pyridin-2,5-diyl, Pyrimidin-2,5-diyl, trans-1,4-Cyclohexylen, bei dem ein oder zwei H-Atome durch CN und/oder $CH_3$ ersetzt sein können, (1,3,4)-Thiadiazol-2,5-diyl oder Haphthalin-2,6-diyl, wobei ein oder mehrere H-Atome durch F, Cl und/oder CN ersetzt sein können;

m, n sind null oder eins, mit der Bedingung, daß die Summe aus m + n gleich null oder eins ist.

Die erfindungsgemäß verwendeten Verbindungen der Formel (I) sind teilweise bekannt und teilweise neu.

Gegenstand der Erfindung sind daher auch Verbindungen der Formel (Ia),

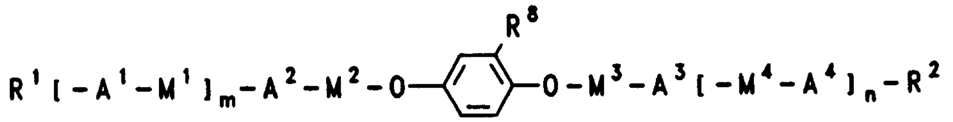

(Ia)

worin die Symbole und Indizes die in Formel (I) angegebenen Bedeutungen haben, mit der Maßgabe, daß

a) bei $R^1$ oder $R^2$ mindestens eine $CH_2$-Gruppe durch Δ oder $-Si(CH_3)_2$-ersetzt ist und/oder $R^1$ oder $R^2$ eine der nachfolgenden chiralen Gruppen ist:

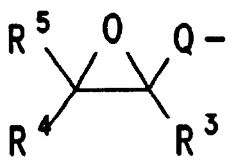

,

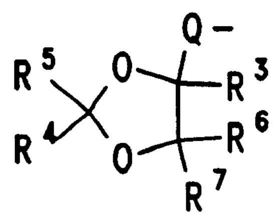

,

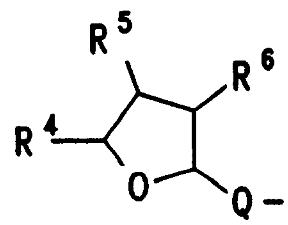

b) und/oder

$A^1$, $A^2$, $A^3$, $A^4$ gleich oder verschieden 1,2-Phenylen, wobei ein oder mehrere H-Atome durch F, Cl und/oder CN ersetzt sein können, 1,3-Phenylen, wobei ein oder mehrere H-Atome durch F, Cl und/oder CN ersetzt sein können, 1,4-Phenylen, wobei 3 oder 4 H-Atome durch F ersetzt sind, Pyrazin-2,3-diyl, wobei ein oder zwei H-Atome durch F ersetzt sind, Pyrazin-2,5-diyl, wobei ein oder zwei H-Atome durch F ersetzt sind, Pyrazin-2,6-diyl, wobei ein oder zwei H-Atome durch F ersetzt sind, Pyridazin-3,4-diyl, wobei ein oder zwei H-Atome durch F ersetzt sind, Pyridazin-3,5-diyl, wobei ein oder zwei H-Atome durch F ersetzt sind, Pyridazin-3,6-diyl, wobei ein oder zwei H-Atome durch F ersetzt sind, Pyridin-2,3-diyl, wobei ein oder mehrere H-Atome durch F ersetzt sind, Pyridin-2,4-diyl, wobei ein oder mehrere H-Atome durch F ersetzt sind, Pyridin-2,5-diyl, wobei ein oder mehrere H-Atome durch F ersetzt sind, Pyridin-2,6-diyl, wobei ein oder mehrere H-Atome durch F ersetzt sind, Pyrimidin-2,5-diyl, wobei ein oder zwei H-Atome durch F ersetzt sind, Pyrimidin-2,6-diyl, wobei ein oder zwei H-Atome durch F ersetzt sind, 1,2-Cyclohexylen, bei dem ein oder mehrere H-Atome durch CN, $CH_3$, F und oder $CF_3$ ersetzt sein können, 1,3-Cyclohexylen, bei dem ein oder mehrere H-Atome durch CN, $CH_3$, F und/oder $CF_3$ ersetzt sein können, 1,4-Cyclohexylen, bei dem ein oder mehrere H-Atome durch F und/oder $CF_3$ ersetzt sind, (1,3,4)-Thiadiazol-2,5-diyl, 1,3-Dioxan-2,5-diyl, 1,3-Dithian-2,5-diyl, 1,3-Thiazol-2,4-diyl, wobei ein H-Atom durch F ersetzt ist, 1,3-Thiazol-1,5-diyl, wobei ein H-Atome durch F ersetzt ist, Thiophen-2,4-diyl, wobei ein oder zwei H-Atome durch F, Cl und/oder CN ersetzt sein können, Thiophen-2,5-diyl, wobei ein oder zwei H-Atome durch F ersetzt sind, Piperazin-1 ,4-diyl, Piperazin-2,5-diyl,

Naphthalin-2,6-diyl, wobei ein oder mehrere H-Atome durch F ersetzt sind, Naphthalin-2,7-diyl, wobei ein oder mehrere H-Atome durch F, Cl und/oder CN ersetzt sein können.

Die Herstellung von erfindungsgemäßen Verbindungen der Formel (I) erfolgt nach an sich literaturbekannten Methoden, wie sie in Standardwerken zur Organischen Synthese, z.B. Houben-Weyl, Methoden der Organischen Chemie, Georg-Thieme-Verlag, Stuttgart, beschrieben werden.

Die Herstellung erfolgt dabei unter Reaktionsbedingungen, die für die genannten Umsetzungen bekannt und geeignet sind. Dabei kann man auch von an sich bekannten, hier nicht näher erwähnten Varianten Gebrauch machen.

Was die Synthese von Molekülen zur Einführung der Gruppe "RAMAMAM" in die Verbindungen der Formel I betrifft, so sei beispielsweise verwiesen auf DE-OS 23 44 732, 24 50 088, 24 29 093, 25 02 904, 26 36 684, 27 01 591 und 27 52 975 für Verbindungen mit 1,4-Cyclohexylen und 1,4-Phenylen-Gruppen; DE-PS 26 41 724 für Verbindungen mit Pyrimidin-2,5-diyl-Gruppen; DE-OS 40 26 223 und EP-OS 03 91 203 für Verbindungen mit Pyridin-2,5-diyl-Gruppen; DE-OS 32 31 462 für Verbindungen mit Pyridazin-3,6-diyl-Gruppen; DE-OS 29 44 905 und 32 27 916 für Verbindungen mit 1,3-Dioxan-2,5-diyl-Gruppen; DD 160 061 für Verbindungen mit 1,3-Dithian-2,5-diyl-Gruppen; US 4,261,652 und 4,219,256 für Verbindungen mit 1,4-Bicyclo-[2,2,2]octan-1,4-diyl-Gruppen.

Für die direkte Verknüpfung von Aromaten und Heteroaromaten sei beispielsweise auf N. Miyaura, T.Yanagi und A. Suzuki in Synthetic Communications 11 (1981), S. 513-519 verwiesen, auf DE-OS 32 01 721 für Verbindungen mit $-CH_2-CH_2-$Brückengliedern und Koji Seto et al. in Liquid Crystals 8 (1990), S. 861-870 für Verbindungen mit $-C\equiv C-$Brückengliedern.

Die Darstellung 2,4-, 2,6- und 3,5-disubstituierter Pyridine, 2,6-disubstituierter Pyrazine, 2,4- und 4,6-disubstituierter Pyrimidine und 3,5-disubstituierter Pyridazine findet sich beispielsweise in den entsprechenden Bänden der Serie "The Chemistry of Heterocyclic Compounds" von A. Weissberger und E.C. Taylor (Herausgeber).

Verbindungen, die zur Einführung des Restes "RAMAMAM" in die Zielmoleküle dienen, können mit substituierten Hydrochinonderivaten der Formel II

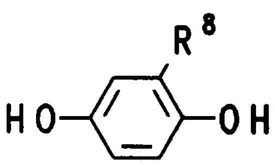

worin $R^8$ die in Formel I angegebene Bedeutung hat, zu Verbindungen der Formel I umgesetzt werden.

Die Verbindungen der Formel II sind teilweise kommerziell erhältlich (z.B. Bromhydrochinon, Methylhydrochinon); sie können auch nach einfachen, dem Fachmann bekannten Verfahren hergestellt werden.

Alkyl-, Fluoralkyl- und Acyl-substituierte Hydrochinone sind durch die Friedel-Crafts-Reaktion darzustellen.

Alkoxy-substituierte Hydrochinone können durch Veretherung von Trihydroxybenzol erhalten werden, Acyloxy-substituierte Hydrochinone können durch Veresterung von Trihydroxybenzol erhalten werden.

Die halogenhaltigen Verbindungen der Formel II sind aus dem Handelsprodukt Bromhydrochinon durch Halogenaustausch darzustellen.

Nitrohydrochinon kann durch Nitrierung von Hydrochinon synthetisiert werden.

Aus Nitrohydrochinon können durch Reduktion zum Amin, Diazotierung und beispielsweise Sandmeyer-Reaktion die Verbindungen mit $R^8$ = F, Cl, SCN, OCH, $N_3$ dargestellt werden.

Die Synthese von Verbindungen der Formel I kann aus den Hydrochinonderivaten der Formel II und geeigneten, den Rest "RAMAMAM" beinhaltenden Verbindungen durch Veretherung oder Veresterung erfolgen.

Eine geeignete Methode zur Veretherung ist beispielsweise die Mitsunobu-Reaktion (siehe z.B. Synthesis 1981, 1).

Durch Veresterung, beispielsweise nach Einhorn oder Schotten-Baumann (siehe z.B. Organikum, 15. Auflage, S. 505f; VEB Deutscher Verlag der Wissenschaften, Berlin 1981) können die Verbindungen hergestellt werden, bei denen das Hydrochinonderivat durch Ester-, Kohlensäureester-, Thioester-oder Thiokohlensäureesterbrücken mit den Resten "RAMAMA" verbunden ist.

Die Ausgangsstoffe können gewünschtenfalls auch in situ gebildet werden und zwar derart, daß man sie aus dem Reaktionsgemisch nicht isoliert, sondern sofort weiter zu den Verbindungen der Formel (I)

umsetzt.

Die erfindungsgemäßen Verbindungen der allgemeinen Formel (I) sind als Komponenten zur Verwendung in ferroelektrischen Flüssigkristallmischungen geeignet, wobei die Verbindungen der allgemeinen Formel (I) nicht notwendigerweise selbst flüssigkristallin sein müssen. Dabei können die FLC-Mischungen 0,01 bis 60 Gew.-%, bevorzugt 0,1 bis 40 Gew.-%, besonders bevorzugt 0,1 bis 20 Gew.-%, an einer oder mehrerer, vorzugsweise 1 bis 5, besonders bevorzugt 1 bis 3 der Verbindungen nach Formel (I) enthalten. Die anderen Bestandteile werden vorzugsweise ausgewählt aus bekannten Verbindungen mit nematischen, cholesterischen und/oder smektischen Phasen; dazu gehören beispielsweise Schiffsche Basen, Biphenyle, Terphenyle, Phenylcyclohexane, Cyclohexylbiphenyle, N-, S- oder O-haltige Heterocyclen, z.B. Pyrimidine, Zimtsäureester, Cholesterinester oder verschieden überbrückte, terminalpolar mehrkernige Ester von p-Alkylbenzoesäuren.

Die erfindungsgemäßen Mischungen wiederum können Anwendung finden in elektrooptischen oder vollständig optischen Elementen, z.B. Anzeigeelementen, Schaltelementen, Lichtmodulatoren, Elementen zur Bildbearbeitung und/oder Signalverarbeitung oder allgemein im Bereich der nichtlineaen Optik.

Die Erfindung wird durch die nachfolgenden Beispiele näher erläutert.

Beispiele

Die Phasenumwandlungstemperaturen werden beim Aufheizen und beim Abkühlen mit Hilfe eines Polarisationsmikroskops anhand der Texturänderungen bestimmt. Die Bestimmung des Schmelzpunkts wird hingegen mit einem DSC-Gerät durchgeführt. Die Angabe der Phasenumwandlungstemperaturen zwischen den Phasen

Isotrop (I)

Nematisch (N bzw. N*)

Smektisch-A ($S_A$)

Smektisch-C ($S_C$ bzw. $S_C$*)

Kristallin (X)

erfolgt in °C, und die Werte stehen zwischen den Phasenbezeichnungen in der Phasenfolge. Es sind zunächst die Phasenübergänge beim Aufheizen in die isotrope Phase angegeben und dann die Phasenübergänge beim Abkühlen, wenn sie von denen des Aufheizens signifikant abweichen.

Synthesebeispiele

Beispiel 1: 3-Propyloxybenzoesäure-4-(3-propylbenzoyloxy)-2-methyl-phenylester

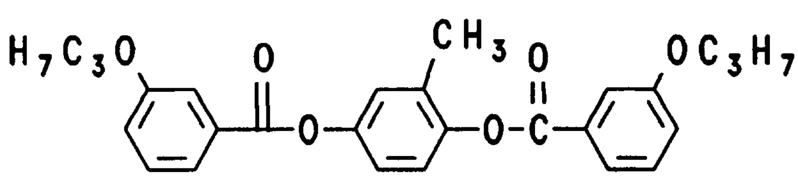

0,68 g (5,5 mmol) Methylhydrochinon und 2 g (11,1 mmol) 3-Propyloxybenzoesäure werden in 50 ml $CH_2Cl_2$ gelöst, und mit 0,1 g Dimethylaminopyridin sowie 2,3 g Dicyclohexylcarbodiimid versetzt. Man rührt 18 Stunden bei Raumtemperatur, zieht das Lösemittel im Vakuum ab und reinigt das Rohprodukt mittels Säulenchromatographie an Kieselgel. Zur weiteren Reinigung wird aus n-Heptan umkristallisiert. Ausbeute: 1,5 g

X 86 $X_1$ 94 I

Analog werden hergestellt:

Beispiel 2: 3-Octyloxybenzoesäure-2-methyl-4(3-octyloxybenzoyloxy)-phenylester

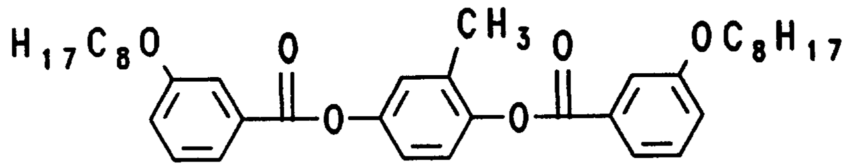

Aus Methyhydrochinon und 3-Octyloxybenzoesäure.
X 72 I

Beispiel 3: 4-Hexyloxybenzoesäure-2-methyl-4-(4-hexyloxybenzoyloxy)-phenylester

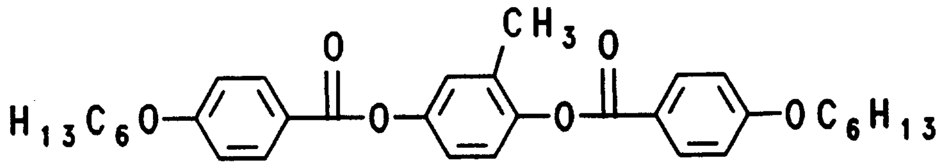

Aus Methylhydrochinon und 4-Hexyloxybenzoesäure.
$X_1$ 67 $X_2$ 87 N 165 I

Beispiel 4: trans-4-Pentylcyclohexylcarbonsäure-2-methyl-4-(trans-4-pentylcyclohexylcarbonyloxy)-phenylester

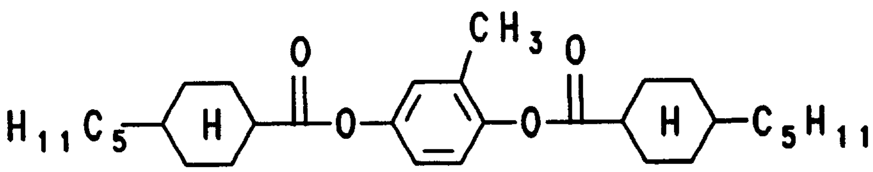

Aus Methylhydrochinon und trans-4-Pentylcyclohexylcarbonsäure.
X 68 $S_1$ 61,5 N 180 I

Beispiel 5: 4-Hexadecyloxybenzoesäure-2-methyl-4-(4-hexadecyloxybenzoyloxy)-phenylester

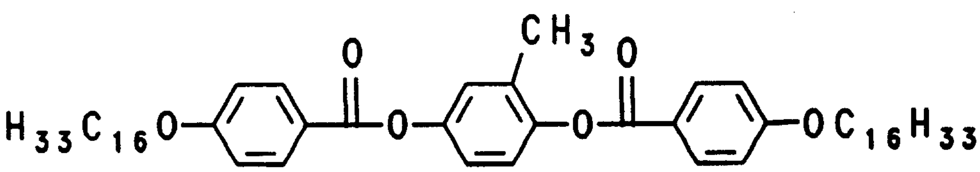

Aus Methylhydrochinon und 4-Hexadecyloxybenzoesäure.
X 86 $S_C$110 N 121-123 I

Beispiel 6: 4-Hexadecyloxybenzoesäure-2-acetyl-4-(4-hexadecyloxybenzoyloxy)-phenylester

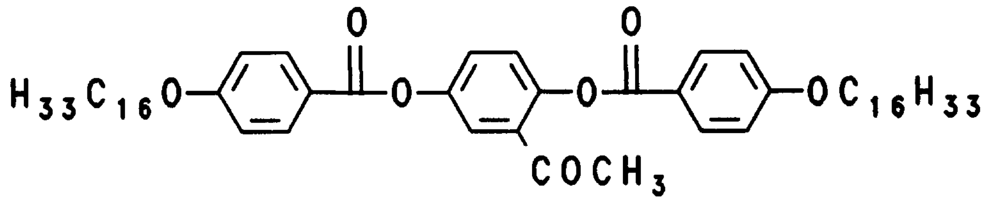

Aus 2-Acetyl-1,4-dihydroxybenzol und 4-Hexadecyloxybenzoesäure.
$X_1$ 101 $X_2$ 93 $S_C$ 129-133 I

Beispiel 7: 4-Dodecyloxybenzoesäure-2-methyl-4-(4-Dodecyloxybenzoyloxy)-phenylester

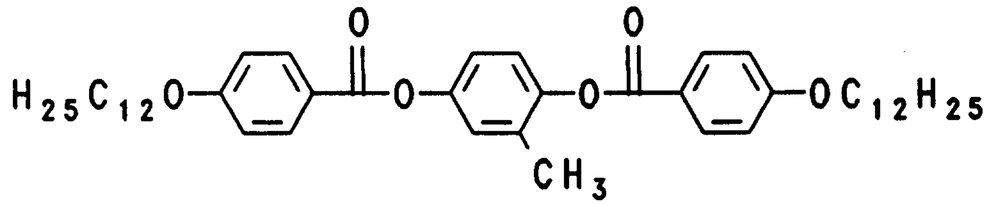

Aus Methylhydrochinon und 4-Dodecyloxybenzoesäure.
X 80 $S_C$ 88,4 N 135,2-136,3 I

Beispiel 8: 4-(3,3,4,4,5,5,6,6,7,7,8,8,8-Tridecafluoroctyloxy)benzoesäure-2-methyl-4-(4-(3,3,4,4,5,5,6,6,7,7,8,8,8-Tridecafluoroctyloxy)-benzoyloxy)-phenylester

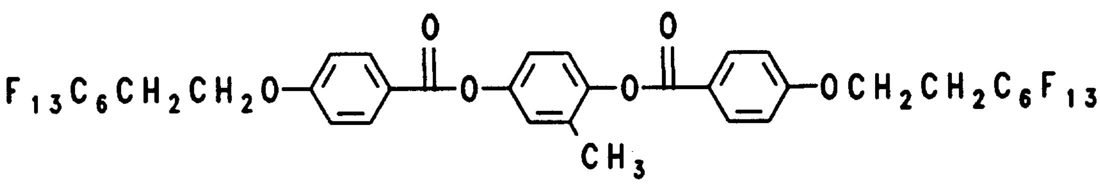

Aus Methylhydrochinon und 4-(3,3,4,4,5,5,6,6,7,7,8,8,8-Tridecafluorocryloxy)-benzoesäure.
X 113 $S_C$ 179 N 170-230 I

Beispiel 9: 2,3,6-Trifluor-4-hexyloxybenzoesäure-2-methyl-4-(2,3,6-trifluor-4-hexyloxybenzoyloxy)-phenylester

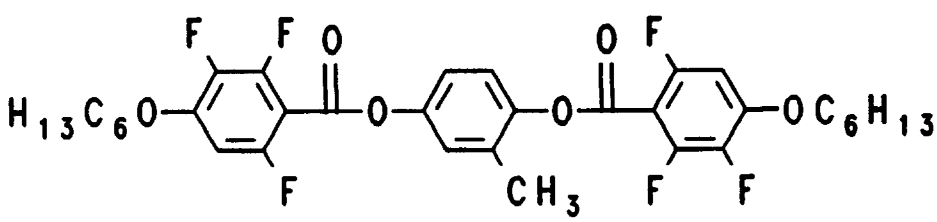

Aus Methylhydrochinon und 2,3,6-Trifluor-4-hexyloxybenzoesäure.
X 74 N 101 I

Beispiel 10: 2,3-Difluor-4-octyloxybenzoesäure-2-methyl-4-(2,3-difluor-4-octyloxybenzoyloxy)-phenylester

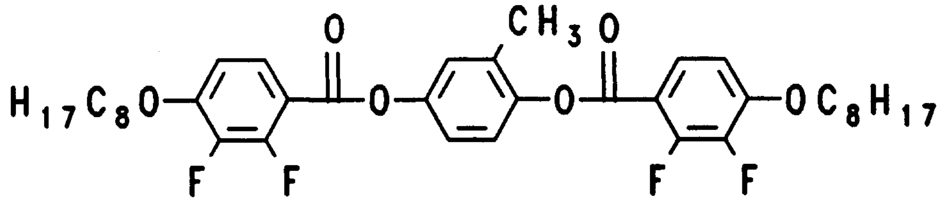

Aus Methylhydrochinon und 2,3-Difluor-4-octyloxybenzoesäure.
X 110 I

Beispiel 11: 4-[4-Butyldimethyl-silanyl)butyloxy]-2,3-difluor-benzoesäure-2-methyl-4-(4-[4-(butyldimethyl-silanyl)butyloxy]-2,3-difluorbenzoyloxy)-phenylester

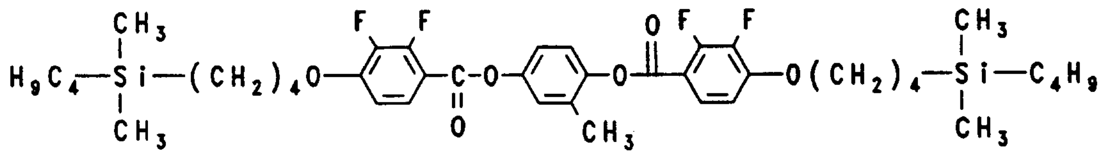

Aus Methylhydrochinon und 4-[4-(Butyldimethyl-silanyl)butyloxy]-2,3-difluorbenzoesäure.
X 82 $S_C$ 58 I

Anwendungsbeispiele

Es wurden folgende FLC-Ausgangsmischungen (enthaltend keine oder eine M3) erfindungsgemäßen Substanzen) verwendet:
M1 mit der Phasenfolge: $S_C$ 79,5 $S_A$ 95,5 N 98 I
und der folgenden Zusammensetzung (Gew.-%):

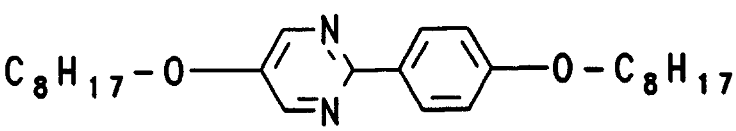

13,75 %

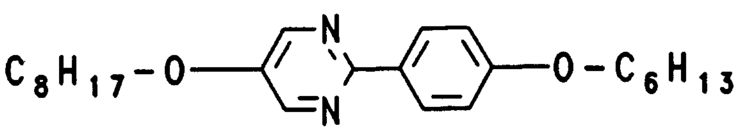

29,80 %

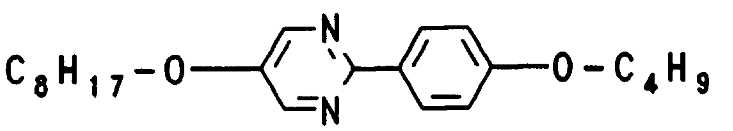

31,43 %

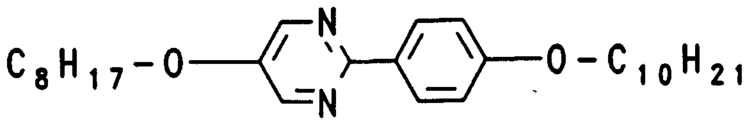

24,99 %

M2 mit der Phasenfolge: $S_C$ 71 $S_A$ 87 I
und der Zusammensetzung (Gew.-%):

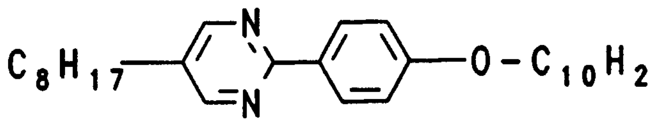

10 %

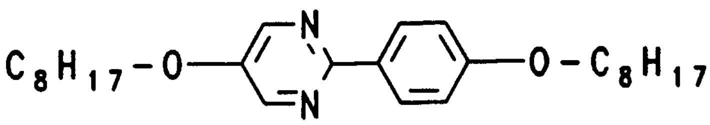

10 %

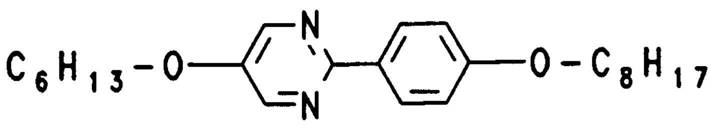

10 %

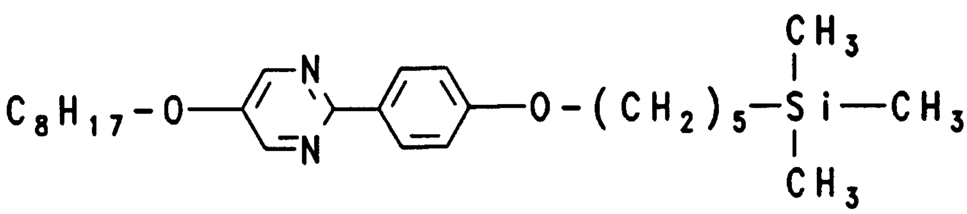

10 %

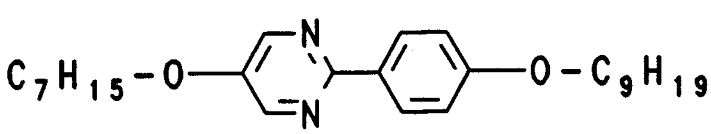

10 %

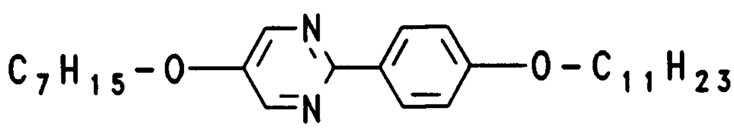

10 %

10 %

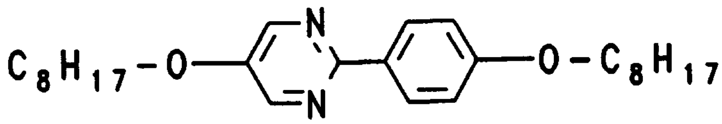

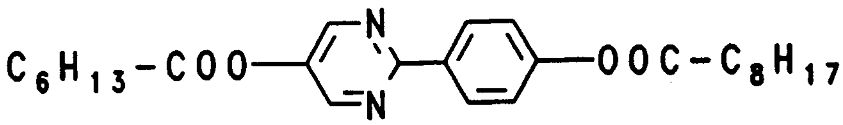

10 %

10 %

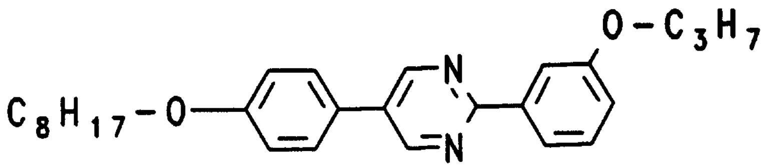

2 %

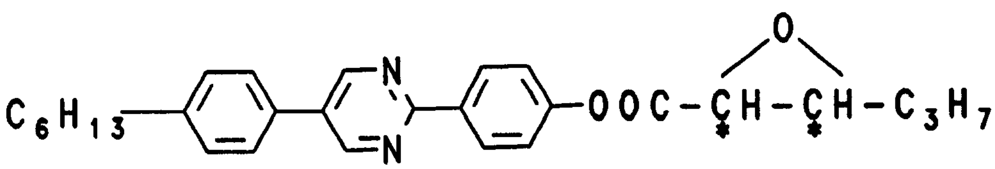

8 %

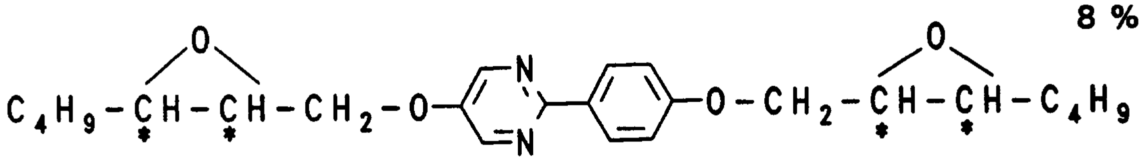

Die Anwendungsbeispiele 1 bis 3 (Tabelle) und 4 belegen die Wirkung der erfindungsgemäßen Substanzen in FLC Mischungen: Die Verbindung Z1

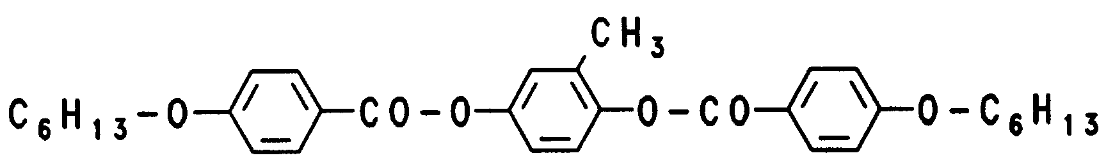

wird in FLC-Ausgangsmischungen M1 und M2 in Mengen Von 1 bis 6 Mol% zugesetzt.

Nur 1 Mol% der Substanz in M1 verbreitert die nematische Phase schon um fast 2 K. Dies zeigt, daß bei Mischungen mit zu schmaler rein nematischer Phase (z.B. mit breiten Biphasenbereichen) durch Zugabe der erfindungsgemäßen Substanz im Prozentbereich eine nematische Phase von genügender Breite erzielt werden kann (Tabelle).

Tabelle: Phasenbreite von Mischung M1 + Z1 in verschiedenen Konzentrationen (MOL-%)

| Anwendungsbeispiel | Mischung | Temperaturen des Phasenübergangs/°C X | $S_C$ | $S_A$ | M | I | Phasenbreite/°C $S_C$ | $S_A$ | N |
|---|---|---|---|---|---|---|---|---|---|
| Vgl. Bsp. | M1 ohne Zusatz | 11.5 | 79.5 | 95.5 | 98 | | 68 | 16 | 2.5 |
| 1 | 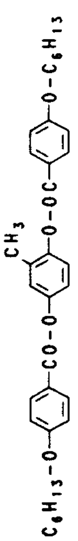 M1 + 1 % Z1 | 11 | 77 | 94 | 98 | | 66 | 17 | 4 |
| 2 | 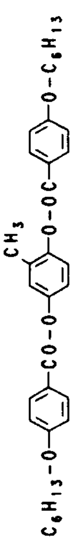 M1 + 2 % Z1 | 11 | 75 | 92 | 98 | | 66 | 17 | 4 |
| 3 | 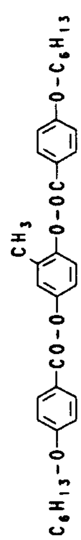 M1 + 4 % Z1 | 11 | 71 | 88 | 97.5 | | 60 | 17 | 9.5 |

Anwendungsbeispiel 4:

Die Mischung M2 weist selbst keine nematische Phase auf. Hier kann durch Zugabe der erfindungsgemäßen Substanz eine nematische Phase erzeugt werden:

| | |
|---|---|
| Phasen ohne: | $S_C$ 71 ° C $S_A$ 87 ° C I |
| Phasen mit 6 % Z1: | $S_C$ 65 ° C $S_A$ 81 ° C N 83 ° C I |

Anwendungsbeispiele 5 bis 10:

Eine Testmischung M3

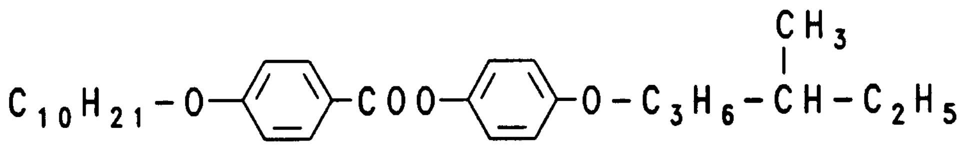

rac 9,0 %

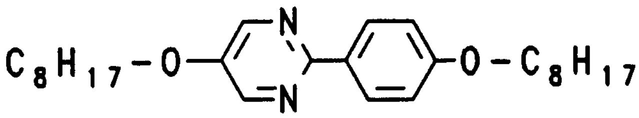

7,8 %

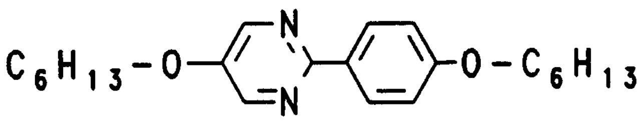

3,5 %

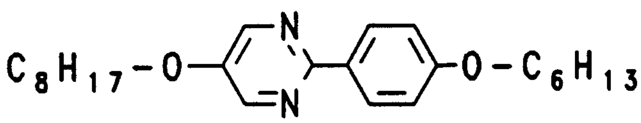

7,2 %

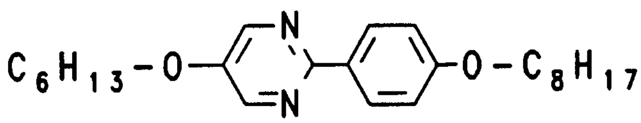

6,6 %

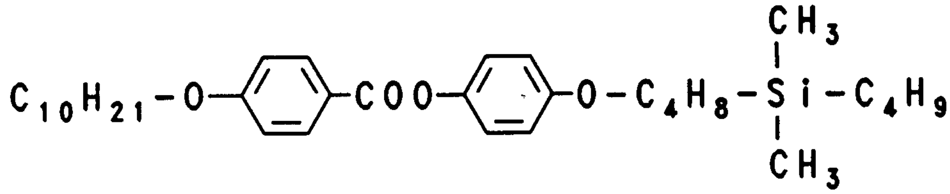

5,3 %

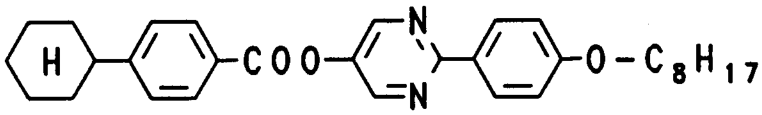

12,7

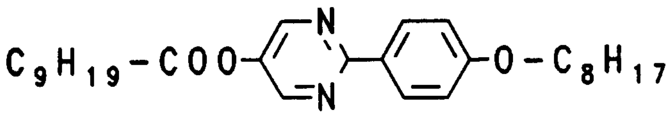

7,7 %

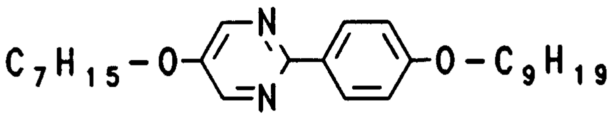

6,4 %

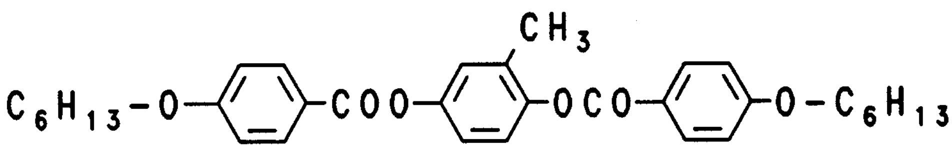

3,0 %

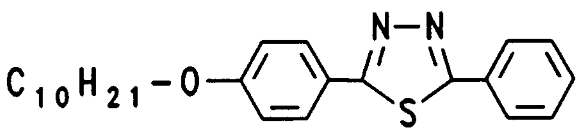

6,6 %

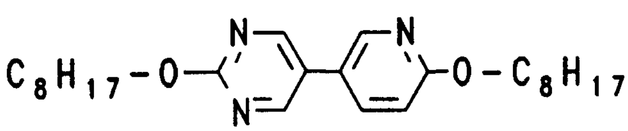

4,0 %

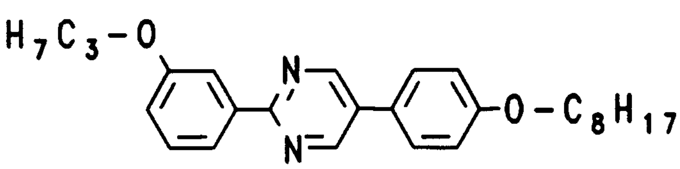

6,3 %

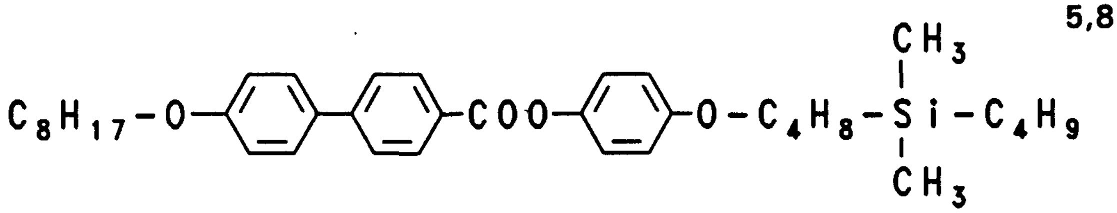

5,8 %

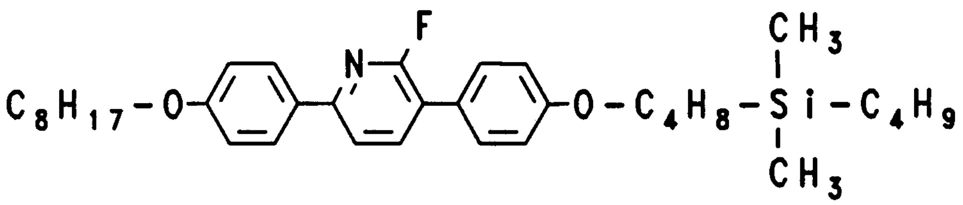

8,1 %

mit den Phasen:

$S_C$ 79,5 ° $S_A$ 90,5 ° N 102 ° I,

wird mit erfindungsgemäßen Verbindungen der Formel (I) versetzt:

5)

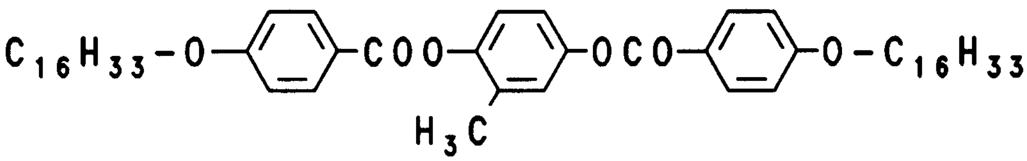

5 Gew.-% in Testmischung M3

$S_C$ 73 ° $S_A$ 85 ° N 102 ° I,

6)

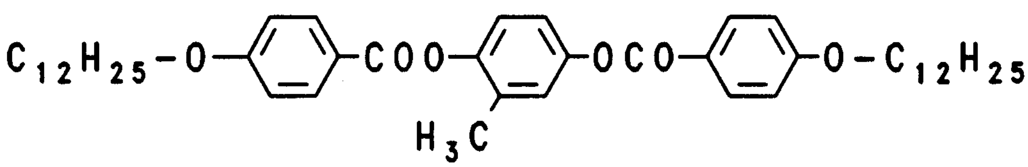

5 Gew.-% in Testmischung M3

$S_C$ 75 ° $S_A$ 84 ° N 102 ° I,

7)

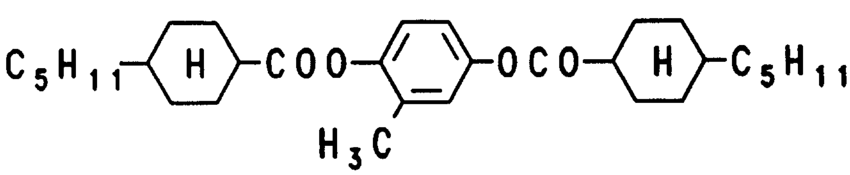

10 Gew.-% in Testmischung M3

$S_C$ 72 ° $S_A$ 86,5 ° N 105 ° I,

8)

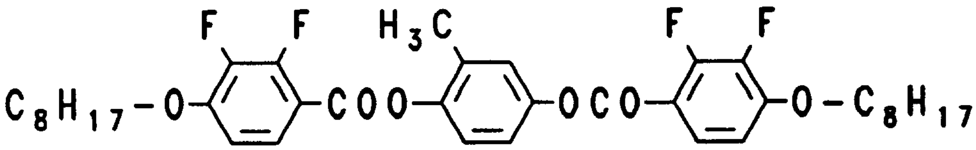

10 Gew.-% in Testmischung M3
$S_C$ 56° N 91° I,

9)

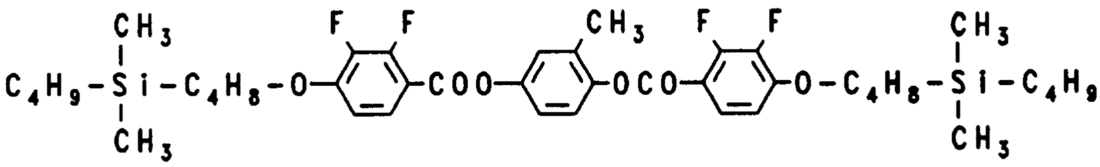

10 Gew.-% in Testmischung M3
$S_C$ 76° N 97° I,

10)

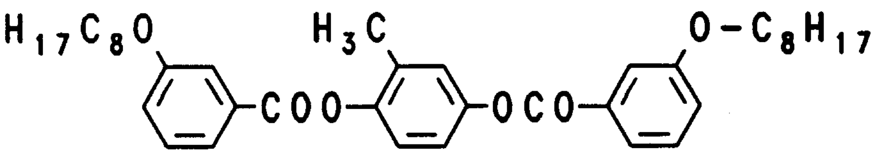

2 Gew.-% in Testmischung M3
$S_C$ 74° $S_A$ 85° N 100° I.

Anwendungsbeispiele 11 bis 17:

Eine Testmischung M4

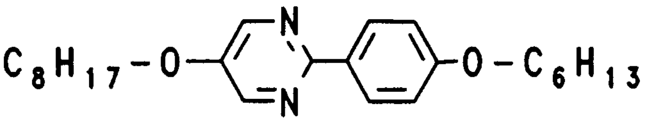

13,1 %

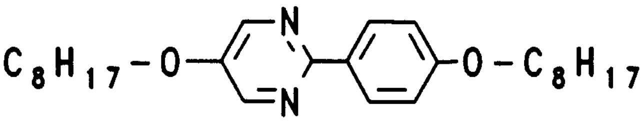

6,5 %

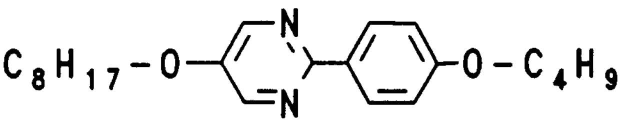

12,8 %

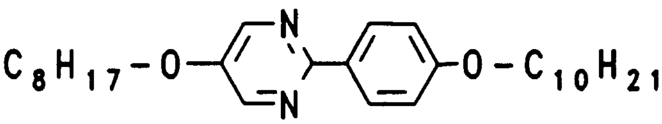

12,6 %

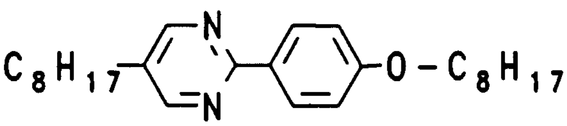

19,4 %

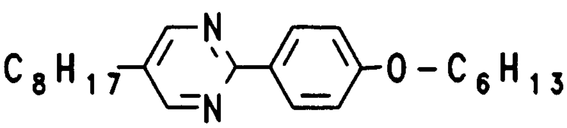

20,1 %

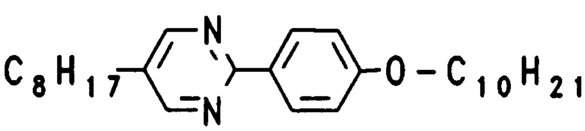

15,5 %

mit den Phasen:

$S_C$ 63,5° $S_A$ 77° N 81,5° I,

wird mit erfindungsgemäßen Verbindungen der Formel (I) versetzt:

11)

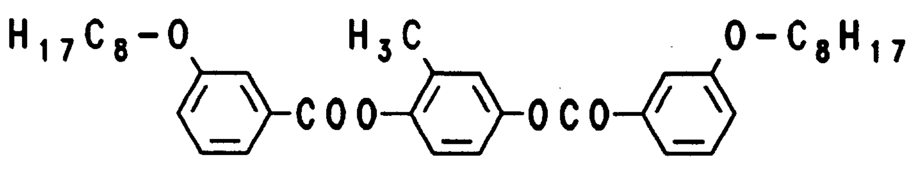

2 Gew.-% in Testmischung M4
$S_C$ 56° $S_A$ 73° N 81° I,
12)

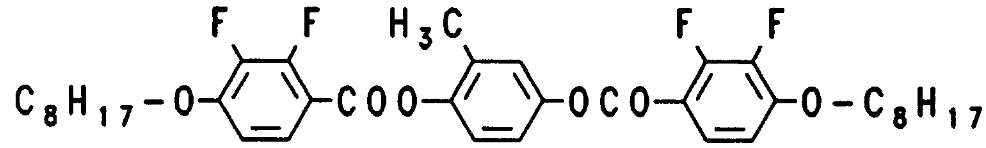

5 Gew.-% in Testmischung M4
$S_C$ 52° $S_A$ 68° N 79° I,
13)

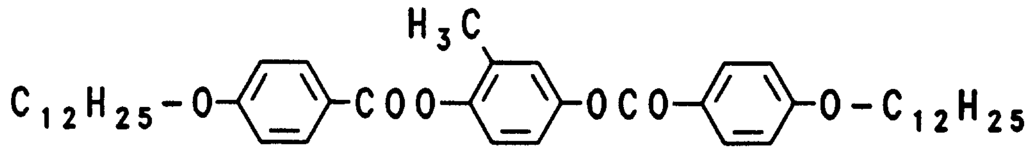

5 Gew.-% in Testmischung M4
$S_C$ 51° $S_A$ 74° N 83° I,
14)

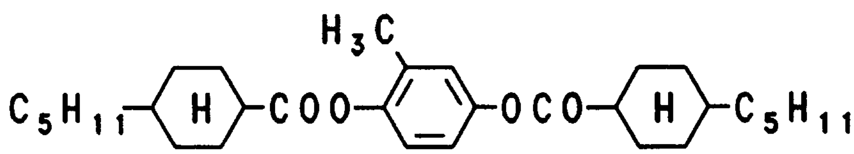

10 Gew.-% in Testmischung M4
$S_C$ 56° $S_A$ 70° N 84° I,
15)

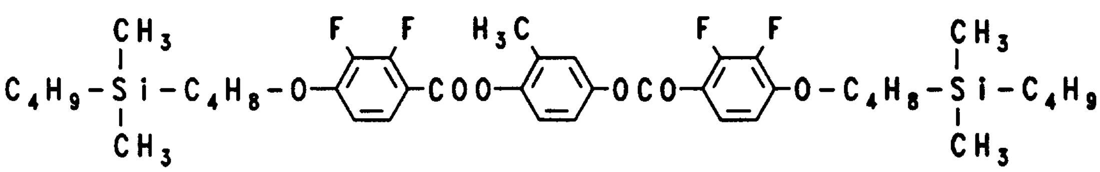

5 Gew.-% in Testmischung M4
$S_C$ 65° $S_A$ 69° N 80° I,
16)

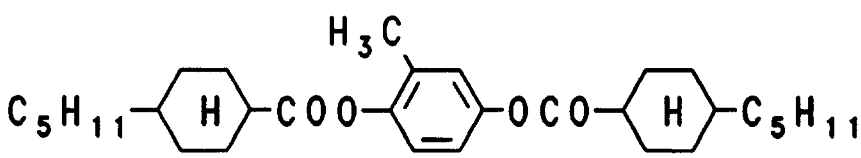

in Testmischung M4
+ 2 Gew.-% obige Substanz $S_C$ 62° $S_A$ 76° N 83° I
+ 6 Gew.-% obige Substanz $S_C$ 59° $S_A$ 73° N 83° I
+ 10 Gew.-% obige Substanz $S_C$ 56° $S_A$ 70° N 84° I

Wie aus obiger Tabelle ersichtlich, kann durch Zugabe auch geringer Mengen der erfindungsgemäßen Verbindungen die nematische Phase stark verbreitert werden.
17)

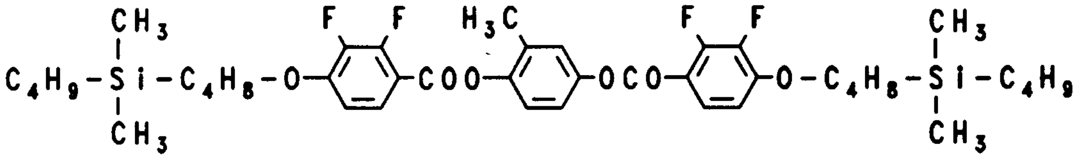

in Testmischung M4
Testmischung M4 $S_C$ 63,5° $S_A$ 77° N 81,5° I
+ 2 Gew.-% obige Substanz $S_C$ 64° $S_A$ 74° N 81,5° I
+ 5 Gew.-% obige Substanz $S_C$ 65° $S_A$ 69° N 80° I
Auch durch geringe Zugabe der erfindungsgemäßen Verbindung kann die nematische Phase stark verbreitert werden.

Anwendungsbeispiel 18

Eine Testmischung M5

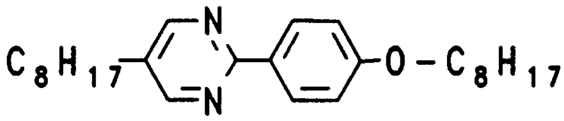

10,7 %

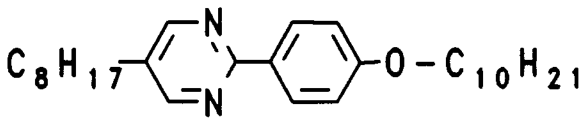

11,0 %

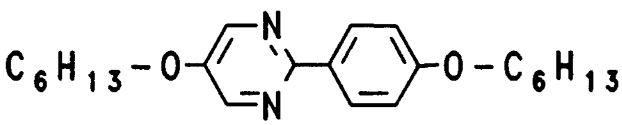

4,9 %

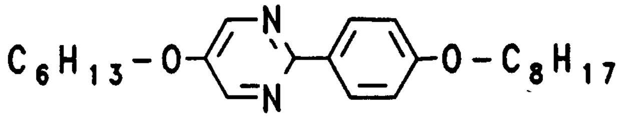

10,5 %

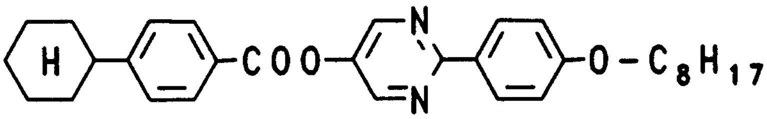

5,3 %

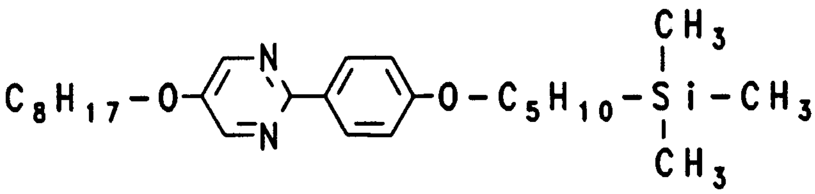

6,0 %

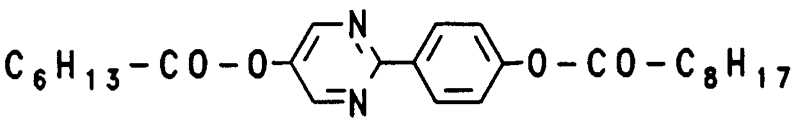

12,0 %

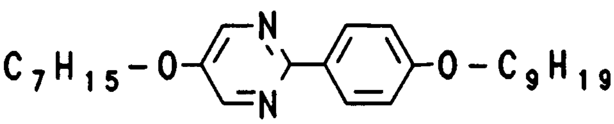

10,7 %

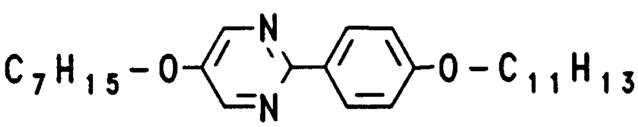

9,6 %

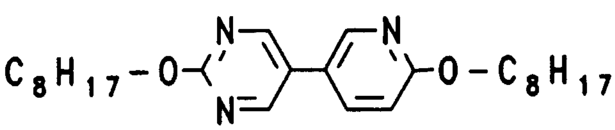

9,6 %

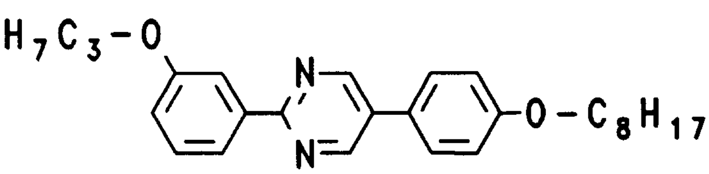

9,7 %

zeigt die Phasenfolge
$S_C$ 68° $S_A$ 88° I

18)

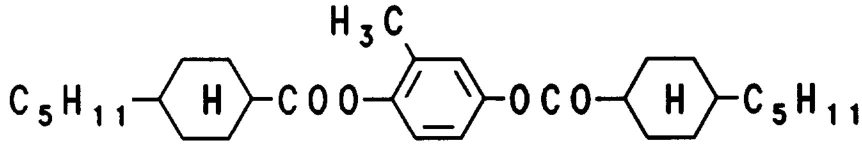

6 Gew.-% in Testmischung M5 zeigt die Phasenfolge

$S_C$ 62° $S_A$ 86° N 89° I

Es kann somit durch Verbindungen obigen Typs auch in Mischungen ohne nematische Phase eine nematische Phase induziert werden.

Alle Anwendungsbeispiele belegen, daß durch die oben beschriebenen Verbindungen nematische Phasen induziert oder bei Mischungen mit schmaler nematischer Phase durch Zusatz geringer Mengen obiger Verbindungen die nematische Phase stark verbreitert werden kann.

**Patentansprüche**

**1.** Ferroelektrische Flüssingkristallmischung, enthaltend mindestens eine Verbindung der Formel I,

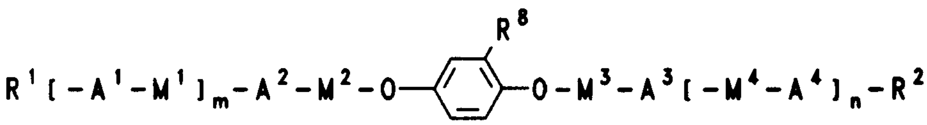

(I)

in der die Symbole und Indizes folgende Bedeutung haben:

$R^1$, $R^2$ sind gleich oder verschieden Wasserstoff oder ein geradkettiger oder verzweigter Alkylrest mit 1 bis 20 C-Atomen (mit oder ohne asymmetrische C-Atome), wobei auch eine oder mehrere $CH_2$-Gruppen durch -O-, -S-, -CO-, -CS-, -C≡C-, Δ, $-Si(CH_3)_2-$, 1,4-Phenylen, trans-1,4-Cyclohexylen oder trans-1,3-Cyclopentylen ersetzt sein können, mit der Maßgabe, daß Sauerstoffatome und/oder Schwefelatome nicht unmittelbar miteinander gebunden sein dürfen, und/oder ein oder mehrere H-Atome des Alkylrestes durch -F, -Cl, -Br, $-OR^3$, -SCN, - OCN oder $-N_3$ substituiert sein können, oder eine der nachfolgenden chiralen Gruppen:

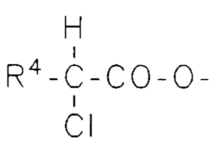 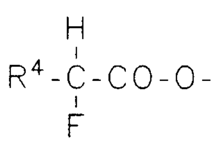 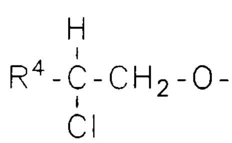 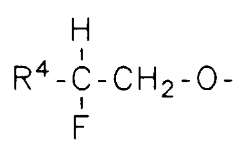

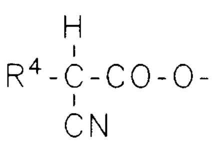 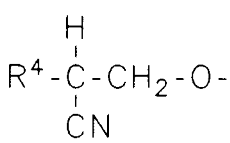 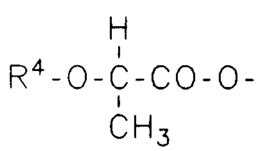 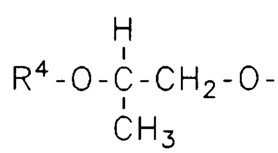

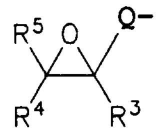
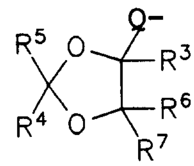
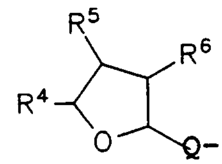

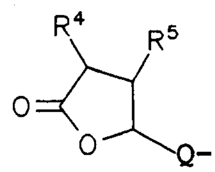
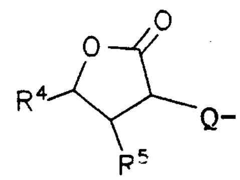
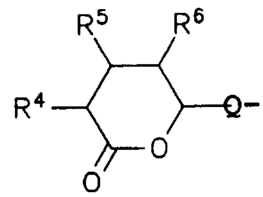

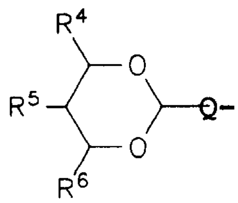
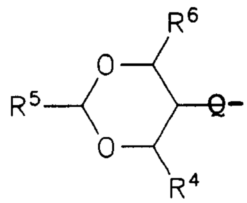
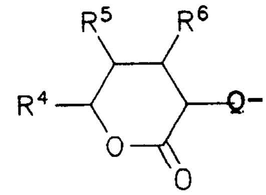

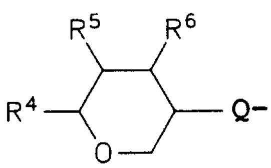
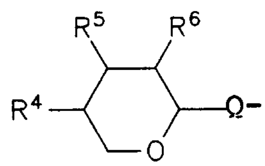
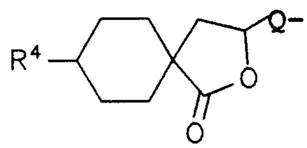

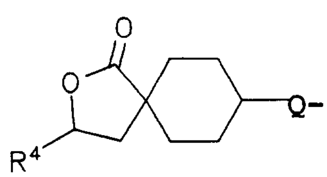
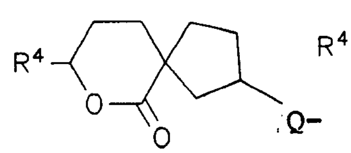
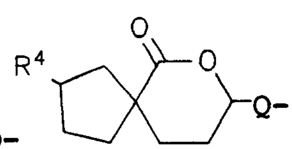

| | |
|---|---|
| $R^3$, $R^4$, $R^5$, $R^6$, $R^7$ | sind gleich oder verschieden Wasserstoff oder ein geradkettiger oder verzweigter Alkylrest mit 1-16 C-Atomen (mit oder ohne asymmetrische C-Atome), wobei auch eine oder mehrere $CH_2$-Gruppen durch - O- oder -CH=CH- ersetzt sein können, mit der Maßgabe, daß Sauerstoffatome nicht unmittelbar miteinander gebunden sein dürfen, und/oder ein oder mehrere H-Atome des Alkylrestes durch - F oder -Cl substituiert sein können; $R^4$ und $R^5$ können zusammen auch $-(CH_2)_4-$ oder $-(CH_2)_5-$ sein, wenn sie an ein Oxiran-, Dioxolan-, Tetrahydrofuran-, Tetrahydropyran-oder Valerolacton-System gebunden sind; |
| $R^8$ | ist $R^3$, Brom, Chlor, Fluor, $-NO_2$, - SCN oder -OCN; |
| Q | ist -CO-O-, -O-CO-, $-CH_2$-O-, $-O-CH_2-$ oder -O-CO-O; |
| $M^1$, $M^4$ | sind gleich oder verschieden -O-, -S-, -CO-, -CO-O-, -O-CO-, -O-CO-O-, $-CH_2$-O-, $-O-CH_2-$, -CH=CH-, -C≡C- oder eine Einfachbindung; |
| $M^2$, $M^3$ | sind gleich oder verschieden -CO-, -CO-O-, oder $-CH_2-$; |
| $A^1$, $A^2$, $A^3$, $A^4$ | sind gleich oder verschieden 1,2-Phenylen, wobei ein oder mehrere H-Atome durch F, Cl und/oder CN ersetzt sein können, 1,3-Phenylen, wobei ein oder mehrere H-Atome durch F, Cl und/oder CN ersetzt sein können, 1,4-Phenylen, wobei ein oder mehrere H-Atome durch F, Cl und/oder CN ersetzt sein können, Pyrazin-2,3-diyl, wobei ein oder zwei H-Atome durch F, Cl und/oder CN ersetzt sein können, Pyrazin-2,5-diyl, wobei ein oder zwei H-Atome durch F, Cl und/oder CN ersetzt sein können, Pyrazin-2,6-diyl, wobei ein oder zwei |

H-Atome durch F, Cl und/oder CN ersetzt sein können, Pyridazin-3,4-diyl, wobei ein oder zwei H-Atome durch F, Cl und/oder CN ersetzt sein können, Pyridazin-3,5-diyl, wobei ein oder zwei H-Atome durch F, Cl und/oder CN ersetzt sein können, Pyridazin-3,6-diyl, wobei ein oder zwei H-Atome durch F, Cl und/oder CN ersetzt sein können, Pyridin-2,3-diyl, wobei ein oder mehrere H-Atome durch F, Cl und/oder CN ersetzt sein können, Pyridin-2,4-diyl, wobei ein oder mehrere H-Atome durch F, Cl und/oder CN ersetzt sein können, Pyridin-2,5-diyl, wobei ein oder mehrere H-Atome durch F, Cl und/oder CN ersetzt sein können, Pyridin-2,6-diyl, wobei ein oder mehrere H-Atome durch F, Cl und/oder CN ersetzt sein können, Pyrimidin-2,5-diyl, wobei ein oder zwei H-Atome durch F, Cl und/oder CN ersetzt sein können, Pyrimidin-2,6-diyl, wobei ein oder zwei H-Atome durch F, Cl und/oder CN ersetzt sein können, 1,2-Cyclohexylen, bei dem ein oder mehrere H-Atome durch CN, $CH_3$, I und oder $CF_3$ ersetzt sein können, 1,3-Cyclohexylen, bei dem ein oder mehrere H-Atome durch CN, $CH_3$, F und/oder $CF_3$ ersetzt sein können, 1,4-Cyclohexylen, bei dem ein oder mehrere H-Atome durch CN, $CH_3$, F und/oder $CF_3$ ersetzt sein können, (1,3,4)-Thiadiazol-2,5-diyl, 1,3-Dioxan-2,5-diyl, 1,3-Dithian-2,5-diyl, 1,3-Thiazol-2,4-diyl, wobei ein H-Atom durch F, Cl und/oder CN ersetzt sein kann, 1,3-Thiazol-1,5-diyl, wobei ein H-Atome durch F, Cl und/oder CN ersetzt sein kann, Thiophen-2,4-diyl, wobei ein oder zwei H-Atome durch F, Cl und/oder CN ersetzt sein können, Thiophen-2,5-diyl, wobei ein oder zwei H-Atome durch F, Cl und/oder CN ersetzt sein können, Piperazin-1,4-diyl, Piperazin-2,5-diyl, Naphthalin-2,6-diyl, wobei ein oder mehrere H-Atome durch F, Cl und/oder CN ersetzt sein können, Naphthalin-2,7-diyl, wobei ein oder mehrere H-Atome durch F, Cl und/oder CN ersetzt sein können, Bicyclo-[2.2.2]octan-1,4-diyl oder 1,3-Dioxaborinan-2,5-diyl;

m, n sind null oder eins.

**2.** Ferroelektrische Flüssigkristallmischung nach Anspruch 1, dadurch gekennzeichnet, daß die Symbole und Indizes der allgemeinen Formel I folgende Bedeutung haben:

$R^1$, $R^2$ sind gleich oder verschieden Wasserstoff oder ein geradkettiger oder verzweigter Alkylrest mit 1 bis 18 C-Atomen (mit oder ohne asymmetrische C-Atome), wobei auch eine oder mehrere $CH_2$-Gruppen durch -O-, -CO-, -C≡C-, Δ , -Si($CH_3$)$_2$- oder trans-1,4-Cyclohexylen ersetzt sein können, mit der Maßgabe, daß Sauerstoffatome nicht unmittelbar miteinander gebunden sein dürfen, und/oder ein oder mehrere H-Atome des Alkylrestes durch - F, -Cl, -$OR^3$, -OCN oder -$N_3$ substituiert sein können, oder eine der nachfolgenden chiralen Gruppen:

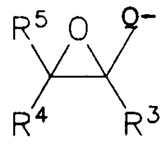
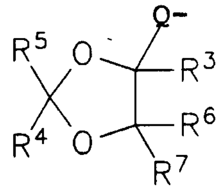
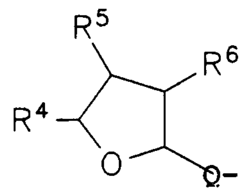

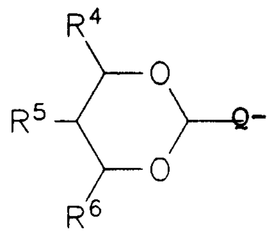
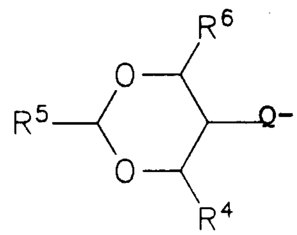
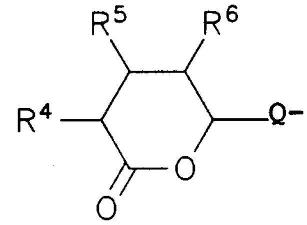

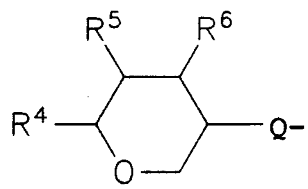
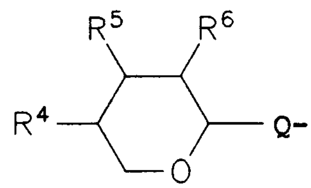
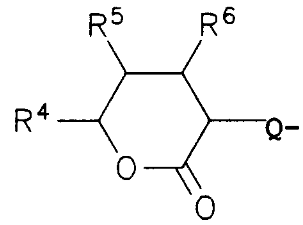

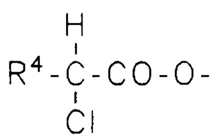
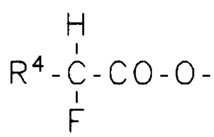
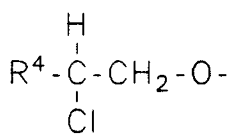
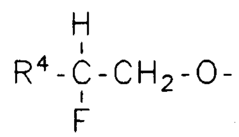

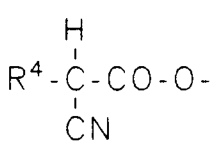
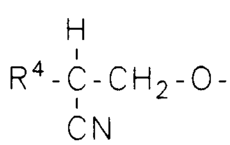
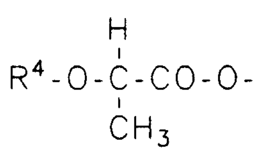
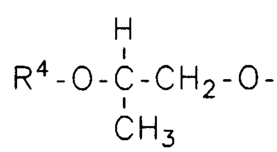

| | |
|---|---|
| $R^3$, $R^4$, $R^5$, $R^6$, $R^7$ | sind gleich oder verschieden Wasserstoff oder ein geradkettiger oder verzweigter Alkylrest mit 1-16 C-Atomen (mit oder ohne asymmetrische C-Atome), wobei auch eine oder mehrere $CH_2$-Gruppen durch - O- oder -CH = CH- ersetzt sein können, mit der Maßgabe, daß Sauerstoffatome nicht unmittelbar miteinander gebunden sein dürfen, und/oder ein oder mehrere H-Atome des Alkylrestes durch - F oder -Cl substituiert sein können; $R^4$ und $R^5$ können zusammen auch $-(CH_2)_4$- oder $-(CH_2)_5$- sein, wenn sie an ein Oxiran-, Dioxolan-, Tetrahydrofuran-, Tetrahydropyran-oder Valerolacton-System gebunden sind; |
| $R^8$ | ist $R^3$, Brom, Chlor, Fluor, $-NO_2$, - SCN oder -OCN; |
| Q | ist -CO-O-, -O-CO-, $-CH_2$-O-, -O-$CH_2$- oder -O-CO-O-; |
| $M^1$, $M^4$ | sind gleich oder verschieden -O-,-CO-, -CO-O-, -O-CO-, -O-CO-O-, -O-CS-O-, $-CH_2$-O-, -O-$CH_2$-, -CH = CH-, -C≡C-oder eine Einfachbindung; |
| $M^2$, $M^3$ | sind gleich oder verschieden -CO-, -CO-O- oder $-CH_2$-; |
| $A^1$, $A^2$, $A^3$, $A^4$ | sind gleich oder verschieden 1,3-Phenylen, wobei ein oder mehrere H-Atome durch F, Cl und/oder CN ersetzt sein können, 1,4-Phenylen, wobei ein oder mehrere H-Atome durch F, Cl und/oder CN ersetzt sein können, Pyrazin-2,5-diyl, wobei ein oder zwei H-Atome durch F, Cl und/oder CN ersetzt sein |

können, Pyrazin-2,6-diyl, wobei ein oder zwei H-Atome durch F, Cl und/oder CN ersetzt sein können, Pyridazin-3,5-diyl, wobei ein oder zwei H-Atome durch F, Cl und/oder CN ersetzt sein können, Pyridazin-3,6-diyl, wobei ein oder zwei H-Atome durch F, Cl und/oder CN ersetzt sein können, Pyridin-2,5-diyl, wobei ein oder mehrere H-Atome durch F, Cl und/oder CN ersetzt sein können, Pyridin-2,6-diyl, wobei ein oder mehrere H-Atome durch F, Cl und/oder CN ersetzt sein können, Pyrimidin-2,5-diyl, wobei ein oder zwei H-Atome durch F, Cl und/oder CN ersetzt sein können, Pyrimidin-2,6-diyl, wobei ein oder zwei H-Atome durch F, Cl und/oder CN ersetzt sein können, 1,2-Cyclohexylen, bei dem ein oder mehrere H-Atome durch CN, $CH_3$, F und oder $CF_3$ ersetzt sein können, cis-1,3-Cyclohexylen, bei dem ein oder mehrere H-Atome durch CN, $CH_3$, F und/oder $CF_3$ ersetzt sein können, trans-1,4-Cyclohexylen, bei dem ein oder mehrere H-Atome durch CN, $CH_3$, F und/oder $CF_3$ ersetzt sein können, (1,3,4)-Thiadiazol-2,5-diyl, 1,3-Dioxan-2,5-diyl, 1,3-Dithian-2,5-diyl, 1,3-Thiazol-2,4-diyl, 1,3-Thiazol-2,5-diyl, wobei ein H-Atom durch F, Cl und/oder CN ersetzt sein kann, Thiophen-2,4-diyl, wobei ein oder zwei H-Atome durch F, Cl und/oder CN ersetzt sein können, Thiophen-2,5-diyl, wobei ein oder zwei H-Atome durch F, Cl und/oder CN ersetzt sein können, Naphthalin-2,6-diyl, wobei ein oder mehrere H-Atome durch F, Cl und/oder CN ersetzt sein können, Naphthalin-2,7-diyl, wobei ein oder mehrere H-Atome durch F, Cl und/oder CN ersetzt sein können oder 1,3-Dioxaborinan-2,5-diyl;

m, n — sind null oder eins.

**3.** Ferroelektrische Flüssigkristallmischung nach Anspruch 1 oder 2, dadurch gekennzeichnet, daß die Symbole und Indizes der allgemeinen Formel I folgende Bedeutung haben:

$R^1$, $R^2$ — sind gleich oder verschieden Wasserstoff oder ein geradkettiger oder verzweigter Alkylrest mit 1 bis 16 C-Atomen (mit oder ohne asymmetrische C-Atome), wobei auch eine, zwei oder drei $-CH_2$-Gruppen durch -O-, -CO-, $\Delta$ , $-Si(CH_3)_2$- oder trans-1,4-Cyclohexylen ersetzt sein können, mit der Maßgabe, daß Sauerstoffatome nicht unmittelbar miteinander gebunden sein dürfen, und/oder ein oder mehrere H-Atome des Alkylrestes durch - F, -Cl oder $-OR^3$ substituiert sein können, oder eine der nachfolgenden chiralen Gruppen:

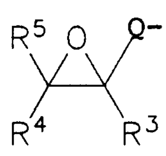
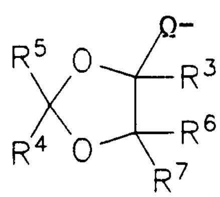
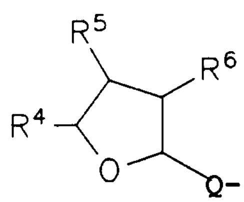

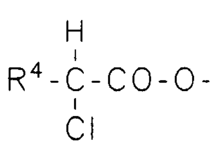
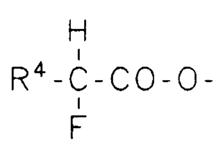
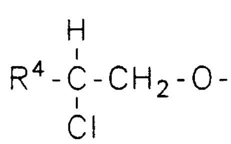
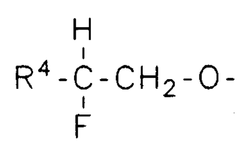

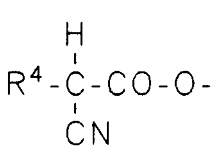
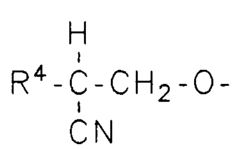
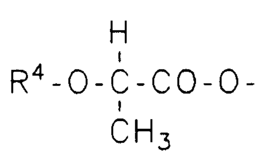
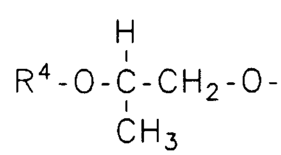

$R^3$, $R^4$, $R^5$, $R^6$, $R^7$ — sind gleich oder verschieden Wasserstoff oder ein geradkettiger oder verzweigter Alkylrest mit 1 bis 14 C-Atomen (mit oder ohne asymmetrische C-Atome), wobei auch eine oder mehrere $-CH_2$-Gruppen durch -O- oder -CH = CH- ersetzt sein können, mit der Maßgabe, daß Sauerstoffatome nicht unmittelbar miteinander gebunden sein dürfen, und/oder ein oder mehrere H-Atome des Alkylrestes durch -F oder -Cl substituiert sein können; $R^4$ und $R^5$ können zusammen auch $-(CH_2)_4$-oder $-(CH_2)_5$- sein, wenn sie an ein Oxiran- oder Dioxolan-System gebunden sind;

$R^8$ ist $R^3$, Brom, Cl, F, $-NO_2$, -SCN oder - OCN;

Q ist -CO-O- oder $-CH_2$-O-;

$M^1$, $M^4$ sind gleich oder verschieden -O-,-CO-, -CO-O-, -O-CO-, -O-CO-O-, $-CH_2$-O-, -O-$CH_2$-, -CH = CH-, oder eine Einfachbindung;

$M^2$, $M^3$ sind gleich oder verschieden -CO-, -CO-O-, $-CH_2$-;

$A^1$, $A^2$, $A^3$, $A^4$ sind gleich oder verschieden 1,4-Phenylen, wobei ein oder zwei H-Atome durch F, Cl und/oder CN ersetzt sein können, Pyrazin-2,5-diyl, wobei ein oder zwei H-Atome durch F, Cl und/oder CN ersetzt sein können, Pyridazin-3,6-diyl, wobei ein oder zwei H-Atome durch F, Cl und/oder CN ersetzt sein können, Pyridin-2,5-diyl, wobei ein oder mehrere H-Atome durch F, Cl und/oder CN ersetzt sein können, Pyrimidin-2,5-diyl, wobei ein oder zwei H-Atome durch F, Cl und/oder CN ersetzt sein können, trans-1,4-Cyclohexylen, bei dem ein oder zwei H-Atome durch CN, $CH_3$, F und/oder $CF_3$ ersetzt sein können, (1,3,4)-Thiadiazol-2,5-diyl, 1,3-Dithian-2,5-diyl, 1,3-Thiazol-2,4-diyl, 1,3-Thiazol-2,5-diyl, Thiophen-2,5-diyl, wobei ein oder zwei H-Atome durch F, Cl und/oder CN ersetzt sein können, Naphthalin-2,6-diyl, wobei ein oder mehrere H-Atome durch F, Cl und/oder CN ersetzt sein können oder 1,3-Dioxan-2,5-diyl;

m, n sind null oder eins mit der Bedingung, daß die Summe m + n kleiner als zwei ist.

**4.** Ferroelektrische Flüssigkristallmischung nach einem oder mehreren der Ansprüche 1 bis 3, dadurch gekennzeichnet, daß die Symbole und Indizes der allgemeinen Formel I folgende Bedeutung haben:

$R^1$, $R^2$ sind gleich oder verschieden Wasserstoff oder ein geradkettiger oder verzweigter Alkylrest mit 1 bis 16 C-Atomen (mit oder ohne asymmetrische C-Atome), wobei auch eine, zwei oder drei $CH_2$-Gruppen durch -O-,-CO-, Δ , $-Si(CH_3)_2$- oder trans-1,4-Cyclohexylen ersetzt sein können, mit der Maßgabe, daß Sauerstoffatome nicht unmittelbar miteinander gebunden sein dürfen, und/oder ein oder mehrere H-Atome des Alkylrestes durch - F oder $-OR^3$ substituiert sein können oder eine der nachfolgenden chiralen Gruppen:

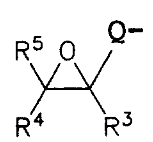
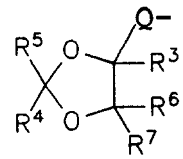

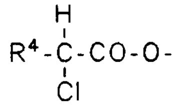
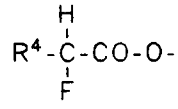
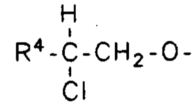
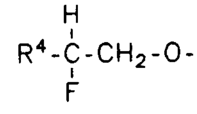

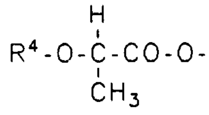
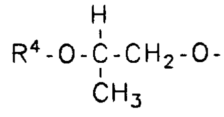

$R^3$, $R^4$, $R^5$, $R^6$, $R^7$ sind gleich oder verschieden Wasserstoff oder ein geradkettiger oder verzweigter Alkylrest mit 1 bis 10 C-Atomen (mit oder ohne asymmetrische C-Atome), wobei auch eine oder zwei $CH_2$-Gruppen durch - O- oder -CH = CH- ersetzt sein können, mit der Maßgabe, daß Sauerstoffatome nicht unmittelbar miteinander gebunden sein dürfen, und/oder ein oder mehrere H-Atome des Alkylrestes durch -F oder -Cl substituiert sein können; $R^4$ und $R^5$ können zusammen auch $-(CH_2)_4-$ oder $-(CH_2)_5$-sein, wenn sie an ein Dioxolan-System gebunden sind;

$R^8$ ist Methyl, Methoxy, Trifluormethyl, Trifluormethoxy, Ethyl, Ethoxy, 1-Propyl, 1-Propyloxy, 2-Propyl, 2-Propyloxy, Acetyl, Trifluoracetyl, $NO_2$, Brom, Chlor oder Fluor;

Q ist -CO-O- oder $-CH_2$-O-;

$M^1$, $M^4$ sind gleich oder verschieden -O-, -CO-, -CO-O-, -O-CO-, -O-CO-O-, $-CH_2$-O-, -O-$CH_2$-, oder eine Einfachbindung;

$M^2$, $M^3$ sind gleich oder verschieden -CO-oder $-CH_2$-;

$A^1$, $A^2$, $A^3$, $A^4$ sind gleich oder verschieden 1,4-Phenylen, wobei ein oder zwei H-Atome durch F, Cl und/oder CN ersetzt sein können, Pyrazin-2,5-diyl, Pyridazin-3,6-diyl, Pyridin-2,5-diyl, Pyrimidin-2,5-diyl, trans-1,4-Cyclohexylen, bei dem ein oder zwei H-Atome durch CN und/oder $CH_3$ ersetzt sein können, (1,3,4)-Thiadiazol-2,5-diyl oder Naphthalin-2,6-diyl, wobei ein oder mehrere H-Atome durch F, Cl und/oder CN ersetzt sein können;

m, n sind null oder eins, mit der Bedingung, daß die Summe aus m + n gleich null oder eins ist.

**5.** Ferroelektrische Flüssigkristallmischung nach einem oder mehreren der Ansprüche 1 bis 4, dadurch gekennzeichnet, daß sie 0,01 bis 60 Gew.-% einer oder mehrerer Verbindungen der Formel (I) enthält.

**6.** Ferroelektrische Flüssigkristallmischung nach Anspruch 5, dadurch gekennzeichnet, daß sie 0,1 bis 40 Gew.-% einer oder mehrerer Verbindungen der Formel (I) enthält.

**7.** Verwendung von Verbindungen der Formel in Anspruch 1 als Komponenten von ferroelektrischen Flüssigkristallmischungen.

**8.** Verwendung nach Anspruch 7, dadurch gekennzeichnet, daß einer ferroelektrischen Flüssigkristallmischung 0,01 bis 60 Gew.-% einer Verbindung der Formel I zugesetzt werden.

**9.** Hydrochinonderivat der Formel (Ia),

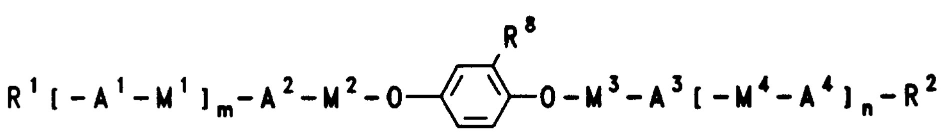

(I a)

worin die Symbole und Indizes die in Formel (I) angegebenen Bedeutungen haben, mit der Maßgabe, daß

a) bei $R^1$ oder $R^2$ mindestens eine $CH_2$-Gruppe durch Δ oder $-Si(CH_3)_2$-ersetzt ist und/oder $R^1$ oder $R^2$ eine der nachfolgenden chiralen Gruppen ist:

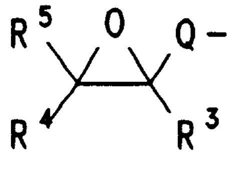

,

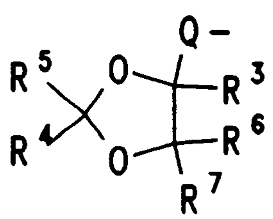

,

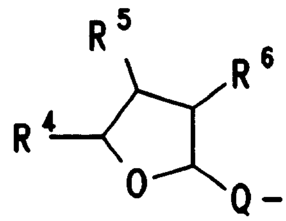

b) und/oder

$A^1$, $A^2$, $A^3$, $A^4$ gleich oder verschieden 1,2-Phenylen, wobei ein oder mehrere H-Atome durch F, Cl und/oder CN ersetzt sein können, 1,3-Phenylen, wobei ein oder mehrere H-Atome durch F, Cl und/oder CN ersetzt sein können, 1,4-Phenylen, wobei 3 oder 4 H-Atome durch F ersetzt sind, Pyrazin-2,3-diyl, wobei ein oder zwei H-Atome durch F ersetzt sind, Pyrazin-2,5-diyl, wobei ein oder zwei H-Atome durch F ersetzt sind, Pyrazin-2,6-diyl, wobei ein oder zwei H-Atome durch F ersetzt sind, Pyridazin-3,4-diyl, wobei ein oder zwei H-Atome durch F ersetzt sind, Pyridazin-3,5-diyl, wobei ein oder zwei H-Atome durch F ersetzt sind, Pyridazin-3,6-diyl, wobei ein oder zwei H-Atome durch F ersetzt sind, Pyridin-2,3-diyl, wobei ein oder mehrere H-Atome durch F ersetzt sind, Pyridin-2,4-diyl, wobei ein oder mehrere H-Atome durch F ersetzt sind, Pyridin-2,5-diyl, wobei ein oder mehrere H-Atome durch F ersetzt sind, Pyridin-2,6-diyl, wobei ein oder mehrere H-Atome durch F ersetzt sind, Pyrimidin-2,5-diyl, wobei ein oder zwei H-Atome durch F ersetzt sind, Pyrimidin-2,6-diyl, wobei ein oder zwei H-Atome durch F ersetzt sind, 1,2-Cyclohexylen, bei dem ein oder mehrere H-Atome durch CN, $CH_3$, F und oder $CF_3$ ersetzt sein können, 1,3-Cyclohexylen, bei dem ein oder mehrere H-Atome durch CN, $CH_3$, F und/oder $CF_3$ ersetzt sein können, 1,4-Cyclohexylen, bei dem ein oder mehrere H-Atome durch F und/oder $CF_3$ ersetzt sind, (1,3,4)-Thiadiazol-2,5-diyl, 1,3-Dioxan-2,5-diyl, 1,3-Dithian-2,5-diyl, 1,3-Thiazol-2,4-diyl, wobei ein H-Atom durch F ersetzt ist, 1,3-Thiazol-1,5-diyl, wobei ein H-Atome durch F ersetzt ist, Thiophen-2,4-diyl, wobei ein oder zwei H-Atome durch F, Cl und/oder CN ersetzt sein können, Thiophen-2,5-diyl, wobei ein oder zwei H-Atome durch F ersetzt sind, Piperazin-1,4-diyl, Piperazin-2,5-diyl, Naphthalin-2,6-diyl, wobei ein oder mehrere H-Atome durch F ersetzt sind, Naphthalin-2,7-diyl, wobei ein oder mehrere H-Atome durch F, Cl und/oder CN ersetzt sein können.

**10.** Elektrooptisches Bauelement, enthaltend eine ferroelektrische Flüssigkristallmischung gemäß einem oder mehreren der Ansprüche 1 bis 6.